# EUROPÄISCHE PATENTANMELDUNG

(11) **EP 3 872 165 A1**
(43) Veröffentlichungstag der Anmeldung: **01.09.2021**
(21) Anmeldenummer: 20160085.5
(22) Anmeldetag: 28.02.2020
(51) Int. Cl.: C12M 3/06, C12M 1/00, C12M 1/34, C12M 1/36

(54) **VERFAHREN ZUM KULTIVIEREN VON ZELLEN**

(71) Anmelder: ibidi GmbH, 82166 Gräfelfing (DE); Fraunhofer-Gesellschaft zur Förderung der angewandten Forschung e.V., 80686 München (DE)
(72) Erfinder: Zantl, Roman, 82166 Gräfelfing (DE); von Guttenberg, Zeno, 82166 Gräfelfing (DE); Baumann, Nina, 82166 Gräfelfing (DE); Walter, Moriz, 88239 Wangen im Allgäu (DE); Traube, Andrea, 72622 Nürtingen (DE)
(74) Vertreter: Grünecker Patent- und Rechtsanwälte PartG mbB

(57) **Zusammenfassung**

Die Erfindung betrifft ein Verfahren zum Kultivieren von Zellen. Die Zellen werden in einem Substrat kultiviert, wobei in dem Substrat, ein Fluidkanalsystem ausgebildet ist, das einen Kultivierungskanal mit einem Kultivierungszugang nach außen, einen Speicherkanal mit einem Speicherzugang nach außen und einen Überführungskanal mit einem Überführungszugang nach außen umfasst, wobei der Kultivierungskanal und der Speicherkanal fluidisch über den Überführungskanal miteinander verbunden sind. Das Verfahren umfasst, Zuführen einer Ausgangszellsuspension durch den Kultivierungszugang in den Kultivierungskanal oder durch den Überführungszugang über den Überführungskanal in den Kultivierungskanal, Züchten einer Zellkultur in dem Kultivierungskanal, Überführen von Zellen der Zellkultur aus dem Kultivierungskanal in den Speicherkanal über den Überführungskanal, wobei das Überführen durch Zuführen einer Überführungsflüssigkeit durch den Kultivierungszugang und Transport der Überführungsflüssigkeit durch den Kultivierungskanal und den Überführungskanal in den Speicherkanal erfolgt, und Speichern und/oder Konzentrieren und/oder Homogenisieren und/oder Verdünnen einer die überführten Zellen enthaltenden Zellsuspension in dem Speicherkanal.

## Beschreibung

Die Erfindung betrifft ein Verfahren zum Kultivieren von Zellen.

Zum Kultivieren von Zellen werden Zellen ausgesät, durch Vermehren der Zellen eine Zellkultur gezüchtet und die Zellen der Zellkultur anschließend weiterverwendet, in der Regel im Anschluss an ein Konzentrieren, Homogenisieren und/oder Verdünnen einer Suspension der gezüchteten Zellen. Die gezüchteten Zellen können dann erneut ausgesät, oder dem System für die weitere Verwendung im Labor entnommen werden.

Entscheidend für die Qualität der durch das Kultivieren erhaltenen Zellen ist, dass der Prozess unter möglichst sterilen Bedingungen durchgeführt wird und dass die einzelnen Schritte zu geeigneten Zeitpunkten erfolgen, da andernfalls Zellen schlecht versorgt werden und gegebenenfalls absterben, beispielsweise, wenn die Zellvermehrung nicht rechtzeitig unterbrochen wird und die älteren Zellen nicht mehr ausreichend versorgt werden.

Daher ist es wünschenswert, einen Prozess zu ermöglichen, der nicht zwingend die Anwesenheit und das Mitwirken eines Menschen zur Durchführung der jeweiligen Prozessschritte erfordert. Bekannt sind in dieser Hinsicht Systeme, bei denen verschiedene über Schläuche verbundene Behälter, in denen jeweils ein Prozessschritt stattfindet, vorgesehen sind, wobei zwischen den Flüssigkeitsbehältern Ventile vorgesehen sind, die zusammen mit Pumpen einen Transport von Flüssigkeiten zwischen den Behältern ermöglichen, so dass der Prozess zumindest teilweise ohne menschlichen Eingriff in das System erfolgen kann.

Das beschriebene Vorgehen ist in verschiedener Hinsicht nicht ideal in der Handhabung. Beispielsweise ist ein relativ aufwändiger Umbau erforderlich, wenn die Behälter, in denen Zellen kultiviert, verarbeitet oder gespeichert werden, ausgetauscht werden sollen. Ein häufiger Austausch ermöglicht jedoch bessere Zellqualität. Außerdem sind die Maßnahmen, die erforderlich sind, um das System in dem die Zellen der Zellkultur kultiviert, verarbeitet, gespeichert und transportiert werden steril zu halten. Es ist daher eine Aufgabe der Erfindung, ein Verfahren zum Kultivieren von Zellen bereitzustellen, das eine bessere Handhabbarkeit ermöglicht.

Die Aufgabe wird durch den Gegenstand des Anspruchs 1 gelöst. Demnach werden die Zellen in einem Substrat kultiviert, wobei in dem Substrat, ein Fluidkanalsystem ausgebildet ist, das einen Kultivierungskanal mit einem Kultivierungszugang nach außen, einen Speicherkanal mit einem Speicherzugang nach außen und einen Überführungskanal mit einem Überführungszugang nach außen umfasst, wobei der Kultivierungskanal und der Speicherkanal fluidisch über den Überführungskanal miteinander verbunden sind. Das Verfahren umfasst ein Zuführen einer Ausgangszellsuspension durch den Kultivierungszugang in den Kultivierungskanal oder durch den Überführungszugang über den Überführungskanal in den Kultivierungskanal, ein Züchten einer Zellkultur der Ausgangszellsuspension in dem Kultivierungskanal, ein Überführen von Zellen der Zellkultur aus dem Kultivierungskanal in den Speicherkanal über den Überführungskanal, wobei das Überführen durch Zuführen einer Überführungsflüssigkeit durch den Kultivierungszugang und Transport der Überführungsflüssigkeit durch den Kultivierungskanal und den Überführungskanal in den Speicherkanal erfolgt, und ein Speichern und/oder Konzentrieren und/oder Homogenisieren und/oder Verdünnen einer die überführten Zellen enthaltenden Zellsuspension in dem Speicherkanal.

Wie daraus ersichtlich wird, finden die wesentlichen Prozessschritte alle in dem Substrat statt. Es gibt also keine gesonderten Prozessierungsbehälter. Ein Austausch aller Prozessbereiche kann einfach durch Austauschen des verwendeten Substrats erfolgen, wobei weniger Schritte erforderlich sind, als in dem bekannten System, weil lediglich ein Element statt mehrere Prozessierungsbehälter ausgetauscht werden und die Anschlüsse nur an dem einen Substrat statt an mehreren Prozessierungsbehälter neu hergestellt werden müssen. Insbesondere müssen keine Verbindungen zwischen den einzelnen Prozessbereichen getrennt und neu hergestellt werden. Dies reduziert also den Zeitaufwand, ohne den Kostenaufwand zu erhöhen, da keine aktiven Komponenten, wie Pumpen oder Ventile ausgetauscht werden müssen. Auch genügt es, ein Element, nämlich das Substrat, durch ein neues zu ersetzen und es müssen nicht mehrere neue Elemente angeschafft werden. Außerdem ist es vergleichsweise einfach, ein bis auf die Zugänge geschlossenes System in einem Substrat steril zu halten.

Im Folgenden werden zum Teil der Kultivierungskanal, der Überführungskanal und der Speicherkanal vereinfacht durch den Oberbegriff "Fluidkanäle" oder "Kanäle" bezeichnet. Ebenso werden im Folgenden zum Teil der Kultivierungszugang, der Überführungszugang und der Speicherzugang vereinfacht durch den Oberbegriff "Zugänge" bezeichnet. Wo eine Unterscheidung nicht erforderlich ist, werden zudem im Folgenden zum Teil vereinfacht die am Verfahren beteiligten Flüssigkeiten, beispielsweise Zellsuspension, Überführungsflüssigkeit, Verdünnungsflüssigkeit oder Spülflüssigkeit, durch den Oberbegriff "Flüssigkeit" bzw. "Flüssigkeiten" bezeichnet.

Bei dem Substrat kann es sich um eines der unten beschriebenen erfindungsgemäßen Substrate handeln. Das Substrat kann zur optischen Untersuchung von in dem Substrat befindlichen Zellen geeignet sein. Insbesondere kann es sich um ein flaches und in den zu untersuchenden Bereichen transparentes Substrat handeln. Insbesondere kann das Substrat in Form eines Mikroskopieträgers ausgebildet sein. Dies ist vorteilhaft, weil vor, während und/oder nach dem Kultivierungsverfahren optische Untersuchungen durchgeführt werden können, beispielsweise die Menge und Eigenschaften der Zellen bzw. Zellkulturen untersucht werden, ohne dabei Zellen zu entnehmen. Eine Entnahme der Zellen für Untersuchungen würde das Ergebnis verfälschen und manche Untersuchungen sind nur in dem Substrat möglich. Somit kann eine genauere Überwachung des Kultivierungsprozesses ermöglicht werden.

Die Zugänge ermöglichen die Zufuhr und Abfuhr von Flüssigkeiten und das Anlegen eines Druckes. Die Zugänge können als Zuläufe und Abläufe des Substrats, insbesondere des jeweiligen Kanals verstanden werden
Mit "nach außen" ist hier der Bereich außerhalb des Substrats gemeint. Der Begriff "Zugang nach außen" bedeutet, dass der Zugang nicht ein Zugang über einen anderen Kanal des Fluidkanalsystems oder einer anderen Struktur im Inneren des Substrats ist. Der Zugang stellt eine unmittelbare Verbindung des Kanals zur Umgebung des Substarts dar. Der Zugang kann beispielsweise eine Öffnung in einer der Oberflächen des Substrats sein.

Der Kultivierungskanal ist an beiden Enden offen, mit einem Ende zu Überführungskanal hin und mit einem Ende nach außen. Der Speicherkanal ist zumindest mit einem Ende nach außen offen und weist darüber hinaus eine Verbindung zum Überführungskanal auf. Diese kann, muss sich aber nicht am anderen Ende des Speicherkanals befinden.

Das Züchten einer Zellkultur erfolgt insbesondere durch Vermehren der Zellen in dem Kultivierungskanal. Der Kultivierungskanal kann eine Wachstumsfläche aufweisen, auf der die erste Zellkultur wächst. Der Kultivierungskanal kann also als Wachstumskanal betrachtet werden.

Das Verfahren kann einen Abtransport der Flüssigkeit der Ausgangszellsuspension aus dem Kultivierungskanal, insbesondere aus dem Fluidkanalsystem, umfassen, während sich die Zellen im Kultivierungskanal befinden, also nach dem Zuführen der Ausgangszellsuspension. Das Abführen kann derart erfolgen, dass die Zellen in dem Kultivierungskanal verbleiben. Dies kann insbesondere erreicht werden, indem das Abführen verzögert erfolgt, nachdem sich die Zellen auf dem Boden des Kultivierungskanals abgesetzt haben und gegebenenfalls daran adhärieren.

Das Verfahren kann ein Zuführen einer Nährflüssigkeit, insbesondere durch den Kultivierungszugang in den Kultivierungskanal oder durch den Überführungszugang über den Überführungskanal in den Kultivierungskanal, umfassen. Das Zuführen der Nährflüssigkeit kann insbesondere während des Züchtens der Zellkultur erfolgen. Optional kann das Verfahren ein anschließendes Abführen der Nährflüssigkeit aus Kultivierungskanal umfassen, insbesondere ein Abführen aus dem Fluidkanalsystem. Das Zuführen und Abführen kann jeweils derart erfolgen, dass die Zellen in dem Kultivierungskanal verbleiben. Das Zuführen und Abführen von Nährflüssigkeit kann während des Züchtens wiederholt durchgeführt werden. So kann eine gute Versorgung der Zellen der Zellkultur mit Nährstoffen sichergestellt werden.

Das Verdünnen einer Zellsuspension, insbesondere der die überführten Zellen enthaltenden Zellsuspension, kann umfassen, dass eine Flüssigkeit durch den Fluidkanal, in dem sich die Zellsuspension befindet, beispielsweise durch den Speicherkanal, transportiert wird und beim Abtransport der Flüssigkeit aus dem Fluidkanal ein Teil der Zellen in dem Fluidkanal verbleibt und ein anderer Teil der Zellen zusammen Flüssigkeit aus dem Fluidkanal, insbesondere aus dem Substrat heraus, transportiert wird. Beispielsweise kann ein Verdünnen im Speicherkanal und/oder im Überführungskanal und/oder gegebenenfalls nach dem Überführen der Zellen aus dem Speicherkanal in einen zweiten Kultivierungskanal vor dem Züchten einer zweiten Zellkultur in dem zweiten Kultivierungskanal erfolgen.

Das Konzentrieren einer Zellsuspension, insbesondere der die überführten Zellen enthaltenden Zellsuspension, kann umfassen, dass Zellen in einem Kanal, beispielsweise dem Speicherkanal, zurückgehalten werden, während Flüssigkeit aus dem Kanal abtransportiert wird. Wenn das Konzentrieren im Speicherkanal erfolgt, kann beispielsweise eine semipermeable Membran an einem der Zugänge des Speicherkanals angeordnet sein. Das Aufkonzentrieren kann alternativ im jeweiligen Kultivierungskanal erfolgen. Dann kann beispielsweise eine semipermeable Membran am Zugang des Kultivierungskanals angeordnet sein. Alternativ oder zusätzlich zum Vorsehen einer Membran kann der Boden des Substrats in dem Bereich, in dem ein Konzentrieren erfolgen soll, beispielsweise im Speicherkanal, eine Strukturierung aufweisen, die Zellen zurück hält. Beispielsweise können die Strukturen ein Sägezahn-Profil aufweisen. Alternativ oder zusätzlich kann die Flüssigkeit mit einer ausreichend niedrigen Fließgeschwindigkeit transportiert werden, die so gewählt ist, dass Zellen nicht weggeschwemmt werden.

Ein Homogenisieren einer Zellsuspension, insbesondere der die überführten Zellen enthaltenden Zellsuspension, kann eine Durchmischung durch eine Bewegung der Zellsuspension, beispielsweise eine oszillierende Bewegung, umfassen. Dies kann durch Anlegen geeigneter Drücke bewirkt werden.

Bei dem oben genannten Verfahren können alle Verfahrensschritte, bei denen Flüssigkeiten in dem Fluidkanalsystem bewegt werden, mittels ausschließlich außerhalb des Substrats angeordneten aktiven Elementen erfolgen.

Die Entnahme der Zellen aus dem System kann über einen fluidischen Ausgang aus dem Substrat erfolgen. Dabei kann ein Teil der Zellen entnommen werden, so dass die Zellkultur im Substrat mit den verbleibenden Zellen aufrechterhalten werden kann. Auf diese Weise stehen Zellen im Labor über längere Zeiträume mit gleichbleibender Qualität zur Verfügung. Im Gegensatz dazu, können die Prozessschritte auch so optimiert werden, dass zu einem einzigen Zeitpunkt eine möglichst große Menge an Zellen zur Verfügung steht, die dann dem System komplett entnommen werden.

Insbesondere können die Schritte automatisch durch Ansteuern der aktiven Elemente erfolgen. So wird ermöglicht, dass mehrere Verfahrensschritte nacheinander ausgeführt werden, ohne dass ein menschliches Eingreifen zum Einleiten des jeweils folgenden Prozessschritts erforderlich ist. Insbesondere ist auch kein unmittelbares eingreifen eines Benutzers in das System erforderlich, beispielsweise zum Pipettieren von Flüssigkeiten, so dass das System während des Verfahrens geschlossen bleibt. So kann besonders hohe Sterilität sichergestellt werden. Außerdem kann so ein kontinuierlicher Prozess sichergestellt werden, der ein gleichmäßiges Wachstum der Zellen ermöglicht.

Das Verfahren kann also eine automatische Durchführung eines mehrstufigen Zellkulturprozesses über mehrere Kultivierungsschritte umfassen.

Insbesondere kann das Verfahren vollständig mit einem in einem Gehäuse angeordneten System durchgeführt werden, beispielsweise mit einem Tischgerät.

Bei den aktiven Elementen kann es sich beispielsweise um Pumpen und/oder Ventile handeln. Das Durchführen der Verfahrensschritte kann insbesondere durch Betätigen der aktiven Komponenten erfolgen. Das Betätigen kann beispielsweise das Öffnen von Ventilen, das Schließen von Ventilen, das Schalten eines Mehrwegeventils, das Anschalten von Pumpen, das Ausschalten von Pumpen und/oder das Einstellen von Betriebsparametern von Pumpen umfassen. Das Schalten eines Mehrwegeventils ermöglicht Transportweg der Flüssigkeit im Mehrwegeventil zu ändern. So kann beispielsweise durch das Schalten Flüssigkeit wahlweise in verschiedene an das Ventil angeschlossene Elemente, beispielsweise Leitungen, eingeleitet werden.

Die Ventile können insbesondere jeweils derart ausgebildet sein, dass sie im geschlossenen Zustand weder Gas noch Flüssigkeit durchlassen.

Das Ansteuern der aktiven Elemente kann mittels einer Steuereinrichtung erfolgen, die die aktiven Elemente ansteuert. Das bedeutet insbesondere, dass die Steuereinrichtung die Betätigung der aktiven Elemente steuert.

Durch das Betätigen der aktiven Komponenten können verschiedene Aspekte eingestellt werden, insbesondere automatisch durch Ansteuern der aktiven Komponenten. Das Einstellen kann insbesondere erfolgen, indem Verbindungen zwischen einem Flüssigkeitsbehälter, in dem Flüssigkeit gelagert wird, und dem Fluidkanalsystem hergestellt und/oder unterbrochen werden, beispielsweise durch, insbesondere automatisches, Öffnen und/oder Schließen von Ventilen und/oder, insbesondere automatisches, Schalten von Mehrwegeventilen, und/oder ein zum Transport der jeweiligen Flüssigkeit in das Fluidkanalsystem geeigneter Druck angelegt wird, beispielsweise durch, insbesondere automatisches, Betätigen von Pumpen. Dabei können die folgenden Aspekte eingestellt werden.

Durch das Betätigen der aktiven Komponenten kann eingestellt werden, welche Flüssigkeit dem Fluidkanalsystem zugeführt wird. Beispielsweise kann mittels Einstellen von Ventilen und/oder Betätigen von Pumpen Flüssigkeit aus einem ausgewählten Speicherbehälter mit der zuzuführenden Flüssigkeit zugeführt werden.

Alternativ oder zusätzlich kann durch das Betätigen der aktiven Komponenten eine Reihenfolge von Flüssigkeiten, die dem Fluidkanalsystem zugeführt werden, eingestellt werden. Beispielsweise kann mittels Einstellen von Ventilen und/oder Betätigen von Pumpen eine erste Flüssigkeit aus einem ausgewählten Speicherbehälter mit der ersten zuzuführenden Flüssigkeit zugeführt werden und im Anschluss mittels Betätigen (Öffnen, Schließen und/oder Schalten) von Ventilen und/oder Betätigen von Pumpen eine zweite Flüssigkeit aus einem ausgewählten Speicherbehälter mit der zweiten zuzuführenden Flüssigkeit zugeführt werden.

Alternativ oder zusätzlich kann durch das Betätigen der aktiven Komponenten eingestellt werden, durch welchen Zugang Flüssigkeit dem Fluidkanalsystem zugeführt wird. Dies kann beispielsweise durch Betätigen von den Zugängen zugeordneten Ventilen erfolgen.

Alternativ oder zusätzlich kann durch das Betätigen der aktiven Komponenten eingestellt werden, durch welchen Zugang Flüssigkeit aus dem Fluidkanalsystem entnommen wird. Dies kann beispielsweise durch Betätigen von den Zugängen zugeordneten Ventilen erfolgen.

Alternativ oder zusätzlich kann durch das Betätigen der aktiven Komponenten der Transportweg von Flüssigkeit im Fluidkanalsystem eingestellt werden. Der Transportweg kann durch Betätigen von Pumpen und Ventilen eingestellt werden. Der Transportweg kann sich beispielsweise dadurch ergeben, wo die Pumpe Druck anlegt und wie die den Zugängen zugeordnete Ventile eingestellt sind, und gegebenenfalls durch die der Geometrie des Fluidkanalsystems. Der Transportweg kann beispielsweise die Richtung des Transports der Flüssigkeit umfassen, insbesondere durch welche Kanäle die Flüssigkeit transportiert wird und in welcher Richtung und Reihenfolge sie sich durch die Kanäle bewegt.

Alternativ oder zusätzlich kann durch das Betätigen der aktiven Komponenten die Fließgeschwindigkeit von Flüssigkeit im Fluidkanalsystem eingestellt werden. Dies kann beispielsweise durch Einstellen des Drucks, den die Pumpen anlegen, erfolgen. Die Fließgeschwindigkeit kann dabei insbesondere auch von den Eigenschaften der Flüssigkeit, beispielsweise Viskosität, und/oder der Geometrie des Fluidkanalsystems und/oder Oberflächeneigenschaften des Fluidkanalsystems abhängen. Insbesondere kann die Fließgeschwindigkeit derart eingestellt werden, dass im Substrat, insbesondere im gesamten System, eine laminare Strömung vorliegt. Dies ermöglicht bessere Kontrolle des Zelltransportes und ist zellschonend.

Alternativ oder zusätzlich kann durch das Betätigen der aktiven Komponenten eingestellt werden, wann Flüssigkeit dem Fluidkanalsystem zugeführt und/oder in dem Fluidkanalsystem weitertransportiert und/oder aus dem Fluidkanalsystem abgeführt wird. Das Einstellen kann erreicht werden, indem die Betätigung der Komponenten zu entsprechenden Zeitpunkten erfolgt.

Insbesondere kann eine automatische Betätigung der aktiven Komponenten zu den entsprechenden Zeitpunkten durch Ansteuern der aktiven Komponenten erfolgen. Wann die Flüssigkeit zugeführt und/oder weitertransportiert und/oder abgeführt wird, kann ausschlaggebend dafür sein, wann ein bestimmter Verfahrensschritt eingeleitet oder beendet wird. Beispielsweise Anfang und Ende des Züchtens und somit auch die Menge der gezüchteten Zellen hängen davon ab, wann die Zellen in den Kultivierungskanal gelangen und daraus entfernt werden. Die Effektivität eines enzymatischen Ablösevorgangs von Zellen der Zellkultur im Kultivierungskanal hängt davon ab, wie lange eine Ablöseflüssigkeit in dem Kultivierungskanal verbleibt.

Die Bewegung der Flüssigkeiten und Zellen im Substrat kann lediglich durch Druckunterschiede im geschlossenen System bei entsprechend geöffneten oder geschlossenen Zugängen eingestellt werden. Durch Einstellen der Druckwerte können die Fließgeschwindigkeiten eingestellt werden.

Das Verfahren kann automatisch erfolgen, indem alle Verfahrensschritte, bei denen Flüssigkeiten in dem Fluidkanalsystem bewegt werden, durch entsprechendes Ansteuern von bzw. der außerhalb des Substrats angeordneten aktiven Komponenten durchgeführt werden. Das Verfahren ermöglicht, einzustellen, dass Zellen sich über einen geeigneten Zeitraum vermehren können, wobei gegebenenfalls die Flüssigkeit im Kultivierungskanal ausgetauscht wird, die Vermehrung zu einem geeigneten Zeitpunkt unterbrochen wird, und die Zellen dann abtransportiert werden.

Gegebenenfalls kann mittels Messeinrichtungen und einem Regelkreis auch eine individuelle Anpassung der Verfahrensschritte für den jeweiligen Einzelfall erfolgen. Daraus ist ersichtlich, dass an sich keine manuellen Schritte erforderlich sind, um den gesamten Kultivierungsprozess durchzuführen. Der Kultivierungsprozess kann also vollautomatisch erfolgen. Alternativ können auch nur Teile des Kultivierungsprozesses automatisch durchgeführt werden.

Bei einer automatischen Durchführung des Verfahrens kann die zeitliche Abfolge der Betätigung aktiver Komponenten und/oder die zu bestimmten Zeitpunkten einzustellenden Werte von an den aktiven Komponenten einzustellenden Parametern ganz oder teilweise im Voraus festgelegt werden. Insbesondere können sie basierend auf der für jeden der Verfahrensschritte erforderlichen Flüssigkeit, Flussrichtung bzw. Transportweg und/oder Fließgeschwindigkeit der Flüssigkeit im Substrat festgelegt werden. Bei den einzustellenden Parametern kann es sich beispielsweise um die Leistung oder den anzulegenden Druck von Pumpen und/oder die Stellung der Ventile handeln.

Alternativ oder zusätzlich kann zumindest ein Teil der zeitlichen Abfolge der Betätigung aktiver Komponenten und/oder der Einstellungen der Werte von an den aktiven Komponenten einzustellenden Parametern nicht im Voraus festgelegt, sondern basierend auf Soll-Werten und gemessenen Ist-Werten von Prozess-Messgrößen, beispielsweise Fließgeschwindigkeit, Durchflussrate, Temperatur, Luftfeuchtigkeit und/oder Zusammensetzung der Luft, geregelt werden. Insbesondere können die Ist-Werte der Messgrößen während des Verfahrens mittels Messeinrichtungen, beispielsweise Durchflusssensoren, Thermometer, Hygrometer oder Detektoren zum Messen des Sauerstoffgehalts und/oder CO₂-Gehalts in der Luft, erfasst werden. Die Regelung kann erfolgen, indem die Ansteuerung der Komponenten derart erfolgt, dass sich die Ist-Werte den Soll-Werten annähern. Das Verfahren kann, insbesondere als Teil der Regelung, umfassen, dass Flüssigkeiten vor dem Zuführen in das Substrat, insbesondere in den Kultivierungskanal, auf eine vorgegebene Temperatur gebracht werden. Insbesondere kann dies aktiv, beispielsweise mittels eines Wärmetauschers, erfolgen.

Das Regeln des Verfahrens unter Verwendung der gemessenen Werte erlaubt es, das Verfahren zu stabilisieren und gleichbleibende Qualität zu gewährleisten.

Das Verfahren kann umfassen, dass, insbesondere während des gesamten Verfahrens, mittels Messeinrichtungen Prozessparameter gemessen werden. Dies ermöglicht eine zuverlässige Inprozesskontrolle. Das Verfahren kann insbesondere umfassen, dass Prozessparameter wie Temperatur, Luftfeuchtigkeit, Sauerstoffgehalt und/oder CO₂-Gehalt im System, insbesondere in der unmittelbaren Umgebung des Substrats, gemessen werden. Wenn das Substrat und/oder das System in einem Gehäuse angeordnet sind, können die Prozessparameter beispielsweise an einer oder mehreren Stellen im Gehäuse erfasst werden.

Das Verfahren kann zudem umfassen, dass die gemessenen Werte gespeichert und/oder zum Steuern von Verfahrensschritten verwendet werden und/oder zur Überwachung des Verfahrens verwendet werden. Letzteres kann beispielsweise umfassen, dass automatisch eine Warnung ausgegeben wird, wenn Abweichungen von vorgegebenen Werten vorliegen. Insbesondere kann das Verfahren einen Zugriff auf eine Referenzdatenbank mit zellspezifischen Daten umfassen. Anhand der zellspezifischen Daten und im Verfahren gemessener Daten können Veränderungen der Physiologie der Zellen erkannt werden. Das Verfahren kann umfassen, dass die gemessenen Werte zur Optimierung der Prozessparameter verwendet werden, insbesondere mittels eines selbstlernenden Systems.

Das Verfahren kann insbesondere derart automatisiert erfolgen, dass anhand von Messwerten das Einleiten nachfolgende Verfahrensschritte automatisch ausgelöst wird. Beispielsweise kann anhand einer automatischen Konfluenzbestimmung automatisch entschieden werden, wann die nächste Zellablösung erfolgt.

Das Substrat kann derart ausgebildet sein, dass darin keine aktiven Komponenten, insbesondere Pumpen und Ventile, und/oder keine Messeinrichtungen und/oder keine Steuereinrichtungen angeordnet sind. Das heißt, dass das Substrat derart ausgebildet sein kann, dass zwischen dem Kultivierungskanal und dem Speicherkanal keine Ventile und/oder Pumpen angeordnet sind. Insbesondere können alle aktiven Komponenten, alle Steuereinrichtungen und alle Messeinrichtungen außerhalb des Substrats angeordnet sein. Es kann sich also um ein rein passives Substrat handeln. Dadurch wird nicht ausgeschlossen, dass sich passive Elemente, beispielsweise Membranen oder passive Filter in dem Substrat befinden. Beispielsweise können an einem oder mehreren der Zugänge semipermeable Membranen angeordnet sein. Insbesondere können semipermeable Membranen derart ausgebildet und angeordnet sein, dass beim Abtransport von Flüssigkeit aus dem Substrat die Zellen unabhängig von der Transportgeschwindigkeit der Flüssigkeit in dem Substrat verbleiben. Solche Membranen können insbesondere an Zugängen von Kanälen angeordnet sein, die zum Homogenisieren und/oder Konzentrieren vorgesehen sind, beispielsweise am Speicherzugang.

Ein passives Substrat, in dem lediglich Kanäle und möglicherweise Membranen angeordnet sind, ist vorteilhaft, da es als preisgünstiges Einwegprodukt bzw. Verbrauchsmaterial hergestellt werden kann und ein Kontaminationsrisiko reduziert ist.

Die Zellen der Zellkultur können enzymatisch und/oder mittels beim Bewegen einer Flüssigkeit in dem Kultivierungskanal entstehende Scherkräfte von den Wänden des Kultivierungskanals abgelöst werden, bevor sie über den Überführungskanal in den Speicherkanal überführt werden. Insbesondere kann dieser Schritt durchgeführt werden, wenn die Zellen beim Züchten der Zellkultur an den Wänden des Kultivierungskanals anhaften, insbesondere anwachsen. Zum enzymatischen Ablösen kann eine enzymhaltige Flüssigkeit, beispielsweise Trypsin, in den Kultivierungskanal transportiert werden. Das Verfahren kann umfassen, dass die enzymhaltige Flüssigkeit für einen vorgegebenen Zeitraum in dem ersten Kultivierungskanal verbleibt und anschließend abtransportiert wird. Die Zellen können ganz oder teilweise mit der enzymhaltigen Flüssigkeit überführt werden oder mittels einer gesonderten Überführungsflüssigkeit überführt werden. Wenn die Zellen mittels Scherkräften von den Wänden abgelöst werden, kann dies durch geeignete Richtung und Fließgeschwindigkeit der Flüssigkeit erzielt werden. Insbesondere kann zum Ablösen von Zellen mittels Scherkräften auch ein bidirektionaler Fluss verwendet werden.

Das Verfahren kann eine optische Untersuchung der Zellen während des Züchtens und/oder während des Ablösens und/oder während die Zellen im Speicherkanal angeordnet sind umfassen. Insbesondere kann die Untersuchung umfassen, dass Zelldichte oder Zellzahl bestimmt wird, insbesondere, die Konfluenz, also Zellzahl pro Fläche.

Die Bestimmung der Zellzahl pro Fläche kann im Kultivierungskanal erfolgen, beispielsweise während des Ablösevorgangs oder im Anschluss daran. Die Bestimmung der Zellzahl kann alternativ oder zusätzlich im Speicherkanal erfolgen bevor die Zellen in einen weiteren Kultivierungskanal überführt werden. Insbesondere können die Zellen nur dann in einen weiteren Kultivierungskanal überführt werden, wenn die Untersuchung ergibt, dass eine geeignete Zelldichte oder Zellzahl vorliegt. Die Aussaat in einem weiteren Kultivierungskanal mit geeigneter Zelldichte oder Zellzahl ermöglicht ein besonders gleichmäßiges Wachstumsverhalten.

Die Analyse der Zellmorphologie kann anhand der optisch aufgenommen Bilder der Zellkultur durchgeführt werden. Dabei können insbesondere KI (künstliche Intelligenz) Algorithmen zum Einsatz kommen. Im Moment sind dafür am besten Deep Learning Algorithmen geeignet, wie sie z.B. vom TensorFlow Modul der Firma Google kostenlos zur Verfügung gestellt werden. Die Morphologie der Zellen kann dazu verwendet werden, den Zustand der Zellen zu evaluieren und automatisiert die Zellkulturparameter anzupassen, um die Zellkultur zu verbessern.

Die optische Untersuchung kann umfassen, dass auf Basis eines mikroskopisch abgebildeten Bereichs der Wachstumsfläche die Konfluenz repräsentativ für die gesamte Wachstumsfläche bestimmt wird. Die optische Untersuchung kann optional das Untersuchen mehrerer Bereiche des Substrats umfassen. Dazu kann insbesondere ein Objektiv einer optischen Messeinrichtung und/oder das Substrat verfahren werden, insbesondere automatisch. Durch das Untersuchen mehrerer Bereiche kann insbesondere eine repräsentative Untersuchung der gesamten Population und deren Qualität erfolgen. Optional kann die optische Untersuchung eine kontrastverstärkendes Verfahren umfassen, beispielsweise unter Verwendung von. Phasenkontrast, Dunkelfeldbeleuchtung, Digitaler Holographischer Mikroskopie (DHM) oder Differentiellem Interferenzkontrast (DIC). Optional könne auch fluoreszenzbasierte Techniken wie z.B. Weitfeldfluoreszenz oder konfokale fluoreszenzscanning Mikroskopie zu Einsatz kommen. Die optische Untersuchung kann umfassen, dass die Zellen automatisch gezählt werden.

Der Kultivierungskanal kann ein erster Kultivierungskanal sein und die Überführungsflüssigkeit kann eine erste Überführungsflüssigkeit sein. Das Fluidkanalsystem kann dann mindestens einen zweiten Kultivierungskanal umfassen, der fluidisch mit dem Überführungskanal verbunden ist. Der zweite Kultivierungskanal und gegebenenfalls zusätzliche Kultivierungskanäle können jeweils eine oder mehrere der im Zusammenhang mit dem (ersten) Kultivierungskanal beschriebenen Eigenschaften aufweisen. Die Kultivierungskanäle können alle in den Überführungskanal münden oder mit diesem anderweitig fluidisch verbunden sein. Insbesondere können mehrere Kultivierungskanäle, insbesondere parallel zueinander, insbesondere nebeneinander, entlang des Überführungskanals angeordnet sein.

Das Verfahren kann ein Überführen, insbesondere nach dem Speichern und/oder Konzentrieren und/oder Homogenisieren und/oder Verdünnen, der Zellen aus dem Speicherkanal über den Überführungskanal in den zweiten Kultivierungskanal mittels einer zweiten Überführungsflüssigkeit und ein Züchten einer weiteren Zellkultur durch Vermehren der Zellen in dem zweiten Kultivierungskanal umfasst. Der zweite Kultivierungskanal kann einen eigenen bzw. separaten Kultivierungszugang aufweisen.

Insbesondere kann der Überführungskanal mit allen Kultivierungskanälen fluidisch verbunden sein und jeder Überführungsschritt von Zellen kann über den Überführungskanal erfolgen. Er stellt also eine zentrale Verbindungsstruktur im Substrat dar.

Der Überführungskanal ermöglicht eine separate Ansteuerung der einzelnen damit verbundenen Kanäle, beispielsweise der Kultivierungskanäle und des bzw. der Speicherkanäle. Zudem kann über den Überführungskanal und den Zugang des Speicherkanals oder des Kultivierungskanals Flüssigkeit durch den Speicherkanal bzw. Kultivierungskanal transportiert werden, ohne dabei die Flüssigkeit durch andere Kanäle transportieren zu müssen. Somit kommen die anderen Kanäle nicht mit der zu transportierenden Flüssigkeit in Kontakt, was Kontaminierungsrisiken reduziert, und wahlweise können die anderen Kanäle gleichzeitig anderweitig genutzt werden, da sie von dem Transport der Flüssigkeit unberührt bleiben.

Die erste und die zweite Überführungsflüssigkeit können die gleiche oder eine andere Stoffzusammensetzung haben. Insbesondere können sie die selbe Stoffzusammensetzung haben und aus dem selben Flüssigkeitsbehälter zugeführt werden.

Der Überführungskanal kann optional auch zum Prozessieren, insbesondere zum Verdünnen von Zellsuspension im Substrat, verwendet werden.

Das Fluidkanalsystem kann zusätzlich zu dem Speicherkanal auch weitere Speicherkanäle aufweisen, die jeweils eine oder mehrere der im Zusammenhang mit dem Speicherkanal beschriebenen Eigenschaften aufweisen können. Die Speicherkanäle können alle in den Überführungskanal münden oder mit diesem anderweitig fluidisch verbunden sein. Beispielhaft können in dem Substrat gleich viele Speicherkanäle und Kultivierungskanäle ausgebildet sein.

Nach dem Züchten der zweiten Zellkultur kann optional ein Überführen von Zellen der zweiten Zellkultur aus dem zweiten Kultivierungskanal in den Speicherkanal oder in einen weiteren Speicherkanal über den Überführungskanal erfolgen. Der zweite Kultivierungskanal kann einen Kultivierungszugang aufweisen und das Überführen kann durch Zuführen einer dritten Überführungsflüssigkeit durch den Kultivierungszugang des zweiten Kultivierungskanals und Transport der dritten Überführungsflüssigkeit durch den zweiten Kultivierungskanal und den Überführungskanal in den Speicherkanal erfolgen.

Die oben beschriebenen Schritte können wiederholt werden, das heißt, es können sukzessive weitere Zellkulturen jeweils in einem weiteren Kultivierungskanal gezüchtet werden, wobei die Zellen dazwischen in den Speicherkanal oder weitere Speicherkanäle überführt werden.

Das Verfahren kann umfassen, dass Zellen aus dem Speicherkanal, insbesondere unmittelbar durch den Speicherzugang oder mittelbar über den Überführungskanal durch den Überführungszugang, entnommen werden, und/oder dass Zellen aus dem Kultivierungskanal, insbesondere unmittelbar durch den Kultivierungszugang oder mittelbar über den Überführungskanal durch den Überführungszugang, entnommen werden. Beispielsweise kann die Entnahme am Ende des Verfahrens erfolgen. Alternativ kann die Entnahme auch einen Zwischenschritt im Verfahren darstellen. Dies ermöglicht beispielsweise, überschüssige Zellen zu entfernen, Zellen für Untersuchungen zu entnehmen, oder die fertig prozessierten Zellen zu entnehmen.

Das Verfahren kann, wie oben beispielhaft für den ersten Kultivierungskanal beschrieben, ein Zuführen in den Fluidkanal, in dem sich Zellen befinden, und/oder Abführen von Nährflüssigkeit aus dem Fluidkanal, insbesondere aus dem Fluidkanalsystem umfassen. Diese Schritte können insbesondere auch wiederholt durchgeführt werden. Mit anderen Worten kann das Verfahren ein Austausch der Nährflüssigkeit in dem jeweiligen Fluidkanal umfassen.

Das Verfahren kann ein Spülen zumindest eines Teils des Fluidkanalsystems und/oder fluidischer Komponenten und/oder aktiver Komponenten und/oder von Flüssigkeitsbehältern des Systems umfassen. Insbesondere kann je zwischen zwei Kultivierungsschritten ein Spülschritt erfolgen, insbesondere ein Spülschritt der außerhalb des Substrats angeordneten Komponenten. Der Spülschritt kann automatisch erfolgen.

Die Erfindung betrifft auch ein Substrat zum Kultivieren von Zellen, insbesondere zum Kultivieren von Zellen mittels eines der oben beschriebenen Verfahren. In dem Substrat ist ein Fluidkanalsystem ausgebildet, das einen Kultivierungskanal zum Züchten einer Zellkultur mit einem Kultivierungszugang nach außen, einen Speicherkanal zum Speichern und/oder Konzentrieren und/oder Homogenisieren und/oder Verdünnen einer die überführten Zellen enthaltenden Zellsuspension mit einem Speicherzugang nach außen und einen Überführungskanal mit einem Überführungszugang nach außen. Der Kultivierungskanal und der Speicherkanal sind fluidisch über den Überführungskanal miteinander verbunden.

Merkmale und Vorteile die oben im Zusammenhang mit dem Verfahren beschrieben wurden, insbesondere hinsichtlich des beim Verfahren verwendeten Substrats, gelten für das Substrat analog.

Das Substrat kann eine Bodenplatte und eine Deckenplatte aufweisen, wobei die Deckenplatte Aussparungen aufweist, die die Kanalstrukturen des Substrats definieren, und mit der Bodenplatte flüssigkeitsdicht verbunden ist.

Bei der Boden- und Deckenplatte kann es sich jeweils um zwei Spritzgussteile, um eine tiefgezogene und eine ebene Folie oder um ein Spritzgussteil und eine ebene oder tiefgezogene Folie handeln. Die Bodenplatte kann zwischen 0.2 µm und 2 mm dick sein. Die Deckenplatte umfassen und die Bodenplatte und Deckenplatte können die gleiche Breite und Länge haben. Die Bodenplatte und Deckenplatte können irreversibel miteinander verbunden sein, insbesondere verklebt, Ultraschall-gebondet oder über Hitze oder ein Lösungsmittel gebondet sein.

Das Substrat kann aus einem, insbesondere biokompatiblen, Kunststoff bestehen. Das Kunststoffmaterial kann beispielsweise Polycarbonat, Polystyrol, Polyethylen, Polyvinylchlorid, Cyclo-olefin Copolymer, Cyclo-olefin Polymer oder Polymethylmethacrylat umfassen.

Das Substrat, insbesondere die Bodenplatte und/oder die Deckenplatte, kann aus Material bestehen, das derart ausgebildet ist, dass eine mikroskopische Untersuchung des Zellwachstums möglich ist. Insbesondere kann die Doppelbrechung des Materials so gering sein, dass die mikroskopische Untersuchung möglich ist und/oder die Fluoreszenz des Materials kann so gering sein, dass eine Untersuchung mit Fluoreszenzmikroskopie möglich ist. Insbesondere kann der Brechungsindex des Materials zwischen 1,2 und 1,6 liegen.

Das Substrat, insbesondere die Bodenplatte, kann aus einem gasdurchlässigen Material bestehen. Dies gewährleistet eine optimale Versorgung der Zellen mit Gasen zu gewährleisten.

Das Substrat kann derart ausgebildet sein, dass zwischen dem Kultivierungskanal und dem Überführungskanal und zwischen dem Überführungskanal und dem Speicherkanal keine aktiven Komponenten, insbesondere keine Ventile und keine Pumpen, angeordnet sind.

Insbesondere kann das Substrat derart ausgebildet sein, dass in dem Substrat keine aktiven Komponenten, insbesondere keine Pumpen und keine Ventile, keine Messeinrichtungen und keine Steuereinrichtungen angeordnet sind. Das Substrat kann ein rein passives Substrat sein, wie oben im Detail erläutert.

Das Fluidkanalsystem kann derart ausgebildet sein, insbesondere eine derartige Form, Anordnung, Orientierung und/oder Verbindung der Kanäle, insbesondere des Kultivierungskanals, des Überführungskanals und des Speicherkanals, und der Zugänge, aufweisen, dass allein durch selektives Öffnen und Schließen der Zugänge und/oder das Anlegen von Druck an das Fluidkanalsystem Flüssigkeiten auf den für den jeweiligen Verfahrensschritt vorgesehenen Transportwegen durch das Fluidkanalsystem bewegt werden und/oder mit für den Verfahrensschritt erforderlichen Fließgeschwindigkeiten durch das Fluidkanalsystem bewegt werden.

Der Kultivierungskanal, der Speicherkanal und der Überführungskanal können im Substrat in einer Ebene angeordnet sein. Bei einem Substrat, mit einer Höhe, die geringer ist als die Länge und die Breite, können die Kanäle insbesondere alle auf einer Höhe angeordnet sein.

Der Durchmesser des Überführungskanals kann kleiner oder gleich dem Durchmesser Kultivierungskanal und/oder des Speicherkanals sein. Maßgeblich ist dabei der Maximaldurchmesser des jeweiligen Kanals.

Eine Mündung des Kultivierungskanals in den Überführungskanal kann entlang des Überführungskanals versetzt zu einer Mündung des Speicherkanals in den Überführungskanal angeordnet sein.

Die Längsachse des Überführungskanals kann in einem Winkel größer oder gleich 50°, insbesondere größer oder gleich 55°, insbesondere größer oder gleich 65°, insbesondere größer oder gleich 75°, insbesondere größer oder gleich 85°, insbesondere im Wesentlichen senkrecht zu der jeweiligen Längsachse des bzw. der Kultivierungskanäle angeordnet sein.

Alternativ oder zusätzlich kann die Längsachse des Überführungskanals in einem Winkel kleiner oder gleich 40°, insbesondere kleiner oder gleich 35°, insbesondere kleiner oder gleich 25°, insbesondere kleiner oder gleich 51°, insbesondere kleiner oder gleich 5°, oder im Wesentlichen parallel zur Längsachse des Speicherkanals angeordnet sein. Der Überführungskanal und der Speicherkanal können, insbesondere in diesem Fall, durch eine Fluidverbindung mit einem Durchmesser kleiner oder gleich dem Durchmesser des Überführungskanals und/oder des Speicherkanals, verbunden sein. Die Fluidverbindung kann insbesondere im Wesentlichen senkrecht zum dem Überführungskanal verlaufen.

Alternativ kann Längsachse des Überführungskanals in einem Winkel größer oder gleich 50°, insbesondere größer oder gleich 55°, insbesondere größer oder gleich 65°, insbesondere größer oder gleich 75°, insbesondere größer oder gleich 85°, insbesondere im Wesentlichen senkrecht zu der Längsachse des Speicherkanals angeordnet sein.

Der Kultivierungskanal kann zu dem Überführungskanal hin verengt sein, wobei die engste Stelle insbesondere einen Durchmesser aufweist, der kleiner oder gleich der Hälfte, insbesondere kleiner oder gleich ein Drittel, insbesondere kleiner oder gleich ein Viertel, des maximalen Durchmessers des Kultivierungskanals ist.

Alternativ oder zusätzlich kann der Speicherkanal zu dem Überführungskanal hin verengt sein, wobei die engste Stelle insbesondere einen Durchmesser aufweist, der kleiner oder gleich der Hälfte, insbesondere kleiner oder gleich ein Drittel, insbesondere kleiner oder gleich ein Viertel, des maximalen Durchmessers des Speicherkanals ist. Diese Verengung ist insbesondere Vorteilhaft, wenn die Längsachse des Überführungskanals in einem Winkel größer oder gleich 50°, insbesondere im Wesentlichen senkrecht zu der Längsachse des Speicherkanals verläuft, weil dann die Verengung eine Abgrenzung der Bereiche ermöglicht.

Mindestens eine Wand des Kultivierungskanals, insbesondere der Boden, kann eine Oberflächenbeschaffenheit des Materials, insbesondere eine Materialeigenschaft und/oder eine strukturelle Eigenschaft, und/oder eine Beschichtung aufweist, die das Anwachsen von Zellen fördert, insbesondere eine hydrophile Beschichtung und/oder eine zelladhärierende Beschichtung.

Alternativ oder zusätzlich kann mindestens eine Wand, insbesondere alle Wände, des Überführungskanals und/oder des Speicherkanals eine Oberflächenbeschaffenheit des Materials, insbesondere eine Materialeigenschaft und/oder eine strukturelle Eigenschaft, und/oder eine Beschichtung aufweisen, die das Adhärieren und/oder Anwachsen von Zellen hindert, insbesondere eine hydrophobe Beschichtung und/oder eine zellabweisende Beschichtung. Eine hydrophobe Beschichtung im Überführungskanal und/oder Speicherkanal kann zudem verhindern, dass sich dort Ablagerungen bilden.

Alternativ oder zusätzlich kann mindestens eine Wand des Kultivierungskanals, insbesondere mindestens ein Teilbereich des Bodens oder der Decke oder der Seitenwände, mit einer Oberflächenmodifizierung versehen sein, die hinsichtlich ihrer physikalischen und/oder chemischen Eigenschaften derart schaltbar ist, dass durch das Schalten eine Ablösung der Zellen von der Oberfläche bewirkt wird, insbesondere mit mindestens einer thermoresponsiven Oberflächen-Modifizierung, insbesondere mit einer Beschichtung mit Poly-N-Isopropylacrylamid (PNIPAM)-basierten Polymeren. In diesem Fall ändern sich die Oberflächeneigenschaften durch Abkühlen der Umgebungstemperatur auf unter 32 °C, so dass sich darauf verankerte Proteine und damit auch die daran haftenden Zellen von der Oberfläche ablösen. Dies kann dafür verwendet werden, dass Zellen mit weniger oder sogar ohne Trypsin oder äquivalente Substanzen von der Oberfläche gelöste werden können.

Alternativ oder zusätzlich kann mindestens eine Wand des Kultivierungskanals, insbesondere mindestens ein Teilbereich des Bodens, mit einer regenerierbaren Oberflächenmodifizierung versehen sein, die insbesondere derart ausgebildet ist, dass die Zelladhäsion veränderbar und/oder aktivierbar und/oder deaktivierbar ist.

Bei Oberflächen mit einer regenerierbaren Oberflächenmodifizierung kann die Zelladhäsion beispielsweise über kompetitive Hemmung (beispielsweise mittels Host/Guest Systemen wie Dendrimer/Peptide, Avidin/Biotin oder Adamantan/Cyclodextrin), über photoschaltbare Bindemotive/Peptide (wie z.B. Nitrophenyl modifizierte Bindemotive), über enzymatischen Verdau einer extrazellulären Matrix (z.B. Collagenasen oder andere Proteasen), über pH-Änderung bzw. Änderung des chemischen Potentials (pH- oder Redox-schaltbare Blockpolymere wie Poly (2-(diisopropylamino)ethyl methacrylat / Poly (2-(methacryloyloxy)ethyl phosphorylcholine Blockpolymer), und/oder über Chelatbildner wie EGTA/EDTA oder His-Tag, veränderbar, aktivierbar und/oder deaktivierbar sein .

Der Vorteil von derartigen regenerierbaren Oberflächenmodifizierungen ist, dass der Wachstumsbereich der Kultivierungskammern mehrfach zum Züchten von Zellkulturen, insbesondere für aufeinanderfolgende Zellkulturen, verwendet werden kann. Dazu reduziert sich der Platzbedarf zum Züchten mehrerer Zellkulturen, da nicht für jede Zellkultur ein eigener Kultivierungskanal vorgesehen sein muss.

Mindestens eine Wand des Kultivierungskanals, insbesondere mindestens ein Teilbereich des Bodens und/oder der Seitenwände, kann eine Strukturierung aufweisen, die derart ausgebildet ist, dass die Wachstumsoberfläche, insbesondere die bei bestimmungsgemäßer Nutzung, mit Flüssigkeit, wie zum Beispiel Zellsuspension, bedeckte und/oder umspülte Oberfläche, erhöht wird. Sie kann insbesondere ein Sägezahnprofil und/oder ein Wellenprofil und/oder eine Lamellenstruktur und/oder faserartige und/oder poröse bzw. schwammartige Bereiche aufweisen. Der Boden des Speicherkanals kann eine Strukturierung aufweisen, beispielsweise ein Sägezahnprofil und/oder ein Wellenprofil, die derart ausgebildet ist, dass die Bewegung von Zellen, die auf dem Boden liegen, mindestens in einer Richtung erschwert, insbesondere verhindert wird. Dies ermöglicht insbesondere ein Aufkonzentrieren der Zellen.

Die Anordnung von hydrophiler Beschichtung im Kultivierungskanal und hydrophober Beschichtung im Überführungskanal unterstützt, zusätzlich zur geometrischen Ausgestaltung der Kanäle, die Abgrenzung zwischen den Kanälen. Die Erfindung betrifft auch ein System umfassend eines der oben beschriebenen Substrate zum Kultivieren von Zellen. Merkmale und Vorteile die oben im Zusammenhang mit dem Verfahren und dem Substrat beschrieben wurden, gelten für das System analog.

System umfassend eines der oben beschriebenen Substrate und mindestens drei außerhalb des Substrats angeordnete Flüssigkeitsbehälter, beispielsweise Speicherbehälter für Prozessflüssigkeiten und/oder Abfallbehälter. Der Kultivierungszugang, der Speicherzugang und der Überführungszugang sind jeweils mit mindestens einem der Flüssigkeitsbehälter fluidisch verbunden. Das System umfasst zudem, insbesondere ansteuerbare, aktive Komponenten, insbesondere Pumpen und/oder Ventile, die derart ausgebildet und angeordnet sind, dass sie bei entsprechender Betätigung, insbesondere Ansteuerung, eine kontrollierte Zufuhr von Flüssigkeiten aus den Flüssigkeitsbehältern in das Fluidkanalsystem und/oder einen kontrollierten Transport der Flüssigkeiten in dem Fluidkanalsystem und/oder eine kontrollierte Abfuhr von Flüssigkeiten aus dem Fluidkanalsystem, insbesondere in den Abfallbehälter, bewirken.

Dabei können alle aktiven Komponenten des Systems außerhalb des Substrats angeordnet sein.

Im bestimmungsgemäßen Gebrauch kann das Substrat horizontal angeordnet sein, insbesondere können die Längsachsen aller Kanäle horizontal angeordnet sein. Insbesondere können alle Kanäle in einer Ebene liegen.

Das System kann insbesondere alle aktiven Komponenten umfassen, die zur automatischen Durchführung des Verfahrens verwendet werden.

Das System kann ein Gehäuse umfassen, in dem das Substrat, die aktiven Komponenten, die Flüssigkeitsbehälter und zumindest ein Teil der Messeinrichtungen angeordnet sind. Der Innenraum des Gehäuses kann einen oder mehrere Prozessräume und einen oder mehrere Lagerräume bereitstellen, die jeweils, insbesondere unabhängig voneinander, klimatisiert sein können. Das Substrat kann dabei in dem Prozessraum angeordnet sein. Speicherbehälter für Prozessflüssigkeiten können in dem Lagerraum angeordnet sein. Dass die Räume klimatisiert sind, kann umfassen, dass die Temperatur im jeweiligen Prozessraum bzw. Lagerraum, insbesondere auch zeitabhängig, eingestellt, insbesondere automatisch geregelt wird. Zudem kann der Sauerstoffgehalt und/oder der CO₂-Gehalt und/oder die Luftfeuchtigkeit eingestellt, insbesondere automatisch geregelt werden. Um die jeweiligen Parameter zu regeln, kann das System Sensoren in dem jeweiligen Prozess- bzw. Lagerraum umfassen, die Temperatur, Sauerstoffgehalt, CO₂-Gehalt und/oder Luftfeuchtigkeit messen.

Das System kann Fluidanschlüsse, beispielsweise Luer-Anschlüsse, umfassen, über die die Kanäle des Substrats fluidisch mit Flüssigkeitsbehältern verbunden sind. Die Anschlüsse können an dem Substrat befestigt oder mit diesem integral ausgebildet sein.

Insbesondere können die Anschlüsse zum sterilen Anschließen ausgebildet sein. Dies gilt auch für Anschlüssen an den Flüssigkeitsbehältern. Dazu können bekannte Sterilanschlüsse verwendet werden. So kann ein steriles abgeschlossenes Fluidsystem bereitgestellt werden und somit gute Qualität der gezüchteten Zellen sichergestellt werden.

Die mindestens drei Flüssigkeitsbehälter können wenigstens zwei Speicherbehälter für Prozessflüssigkeiten, beispielsweise für eine Ausgangszellsuspension und für eine Überführungsflüssigkeit, und einen Abfallbehälter umfassen.

Das System kann auch mehrere gleiche oder verschiedene Substrate umfassen. Dies ermöglicht eine hohe Flexibilität hinsichtlich Durchsatz und parallel kultivierbarer Zelllinien.

Das System kann mehrere Leitungen, beispielsweise in Form von Schläuchen, umfassen, über die das Substrat mit den Flüssigkeitsbehältern verbunden ist. Jeder der Zugänge kann mit mindestens einer der Leitungen verbunden sein.

Der Kultivierungszugang kann mit einem Ventil verbunden sein, das zum Herstellen einer Fluidverbindung zwischen dem Kultivierungskanal und mindestens einem der Speicherbehälter und zum Herstellen einer Fluidverbindung zwischen dem Kultivierungskanal und dem Abfallbehälter ausgebildet ist. Alternativ kann der Kultivierungszugang mit zwei Ventilen verbunden sein, von denen eines zum Herstellen einer Fluidverbindung zwischen dem Kultivierungskanal und mindestens einem der Speicherbehälter und das andere zum Herstellen einer Fluidverbindung zwischen dem Kultivierungskanal und dem Abfallbehälter ausgebildet ist.

Alternativ oder zusätzlich kann der Speicherzugang mit einem Ventil verbunden sein, das zum Herstellen einer Fluidverbindung zwischen dem Speicherkanal und dem Abfallbehälter ausgebildet ist, und insbesondere zum Herstellen einer Fluidverbindung zwischen dem Speicherkanal und mindestens einem der Speicherbehälter.

Alternativ oder zusätzlich kann der Überführungszugang mit einem Ventil verbunden sein, das zum Herstellen einer Fluidverbindung zwischen dem Überführungskanal und mindestens einem der Speicherbehälter und zum Herstellen einer Fluidverbindung zwischen dem Überführungskanal und dem Abfallbehälter ausgebildet ist. Insbesondere kann das Ventil zusätzlich zum Herstellen einer Fluidverbindung zwischen dem Überführungskanal und mindestens einem der Speicherbehälter ausgebildet sein. Alternativ kann der Überführungszugang mit einem weiteren Ventil verbunden sein, wobei das weitere Ventil zum Herstellen einer Fluidverbindung zwischen dem Überführungskanal und mindestens einem der Speicherbehälter ausgebildet ist.

Dabei ist mit einer Verbindung zwischen einem Zugang und einem Ventil eine Verbindung gemeint, die nicht über andere Kanäle des Fluidkanalsystems erfolgt, hier als direkte Verbindung bezeichnet. Dazu kann ein dem jeweiligen Zugang zugeordneter Anschluss vorgesehen sein, beispielsweise ein Luer-Anschluss.

Die Verbindung kann jeweils insbesondere mit einem Mehrwegeventil oder mit einem Sperrventil, insbesondere mit mindestens zwei parallel geschalteten Sperrventilen, herstellbar sein.

Das Herstellen einer Fluidverbindung mit einem der Speicherbehälter für Prozessflüssigkeiten kann ein Herstellen einer Fluidverbindung mit einer mit den Speicherbehältern verbundenen Leitung umfassen, wobei jeder der Speicherbehälter, insbesondere über ein Sperrventil, mit der Leitung verbunden ist.

Das System kann mindestens eine außerhalb des Substrats angeordnete Messeinrichtung, die zum Messen von Prozess-Messgrößen, insbesondere Fließgeschwindigkeit und/oder Durchflussrate und/oder Temperatur, ausgebildet ist, und/oder eine außerhalb des Substrats angeordnete Messeinrichtung, die zur optischen Untersuchung der Zellen im Substrat ausgebildet ist. Alle Messeinrichtungen können dabei, wie oben im Detail erläutert, außerhalb des Substrats angeordnet sein.

Das System kann zusätzlich zu der Messeinrichtung auch eine Speichereinrichtung umfassen, die zum Empfangen und Speichern von Daten von der Messeinrichtung ausgebildet ist. Die Daten können beispielsweise für Dokumentation und Analyse des Kultivierungsverfahren verwendet werden.

Das System kann alternativ oder zusätzlich eine Steuereinrichtung umfassen, die dazu ausgebildet ist, die aktiven Komponenten derart zu steuern, dass eines der oben beschriebenen Verfahren, insbesondere automatisch, durchgeführt wird. Die Steuereinrichtung kann dazu ausgebildet sein, die im Verfahren beschriebenen Steuer- und/oder Regelschritte durchzuführen.

Die Steuereinrichtung kann über eine Datenverbindung mit der mindestens einen Messeinrichtung zum Messen von Prozess-Messgrößen und/oder mit der Messeinrichtung zur optischen Untersuchung der Zellen verbunden sein.

Die Steuereinrichtung kann insbesondere ausgebildet sein, Werte von an den aktiven Komponenten einzustellenden Parametern basierend auf Soll-Werten und gemessenen Ist-Werten von Prozess-Messgrößen zu regeln, die mittels der Messeinrichtung zum Messen von Prozess-Messgrößen erfasst werden.

Die Erfindung betrifft auch eine Verwendung eines der oben beschriebenen Substrate oder Systeme zur Durchführung eines der oben beschriebenen Verfahren.

Weitere Merkmale und Vorteile werden nachfolgend anhand der beispielhaften Figuren erläutert. Dabei zeigt:
Figur 1 eine schematische, nicht maßstabsgetreue Draufsicht auf ein Substrat einer ersten Ausführungsform;
Figur 2 eine schematische, nicht maßstabsgetreue Draufsicht auf ein Substrat einer zweiten Ausführungsform;
Figur 3 eine schematische, nicht maßstabsgetreue Draufsicht auf ein Substrat einer dritten Ausführungsform;
Figur 4 eine schematische, nicht maßstabsgetreue Draufsicht auf ein Substrat einer vierten Ausführungsform;
Figur 5 eine schematische, nicht maßstabsgetreue Darstellung eines Systems einer fünften Ausführungsform am Beispiel eines Substrats der ersten Ausführungsform;
Figur 6 eine schematische, nicht maßstabsgetreue Darstellung eines Systems einer sechsten Ausführungsform am Beispiel eines Substrats der zweiten Ausführungsform;
Figur 7 eine schematische, nicht maßstabsgetreue Darstellung eines Systems einer siebten Ausführungsform am Beispiel eines Substrats der dritten Ausführungsform;
Figur 8 eine schematische, nicht maßstabsgetreue Darstellung eines Systems einer siebten Ausführungsform am Beispiel eines Substrats der vierten Ausführungsform;
Figur 9 eine schematische, nicht maßstabsgetreue Darstellung eines Systems einer achten Ausführungsform.

Im Folgenden und in den Figuren werden in den verschiedenen Ausführungsbeispielen, sofern nicht anders spezifiziert die gleichen Bezugszeichen für gleiche oder entsprechende Elemente verwendet.

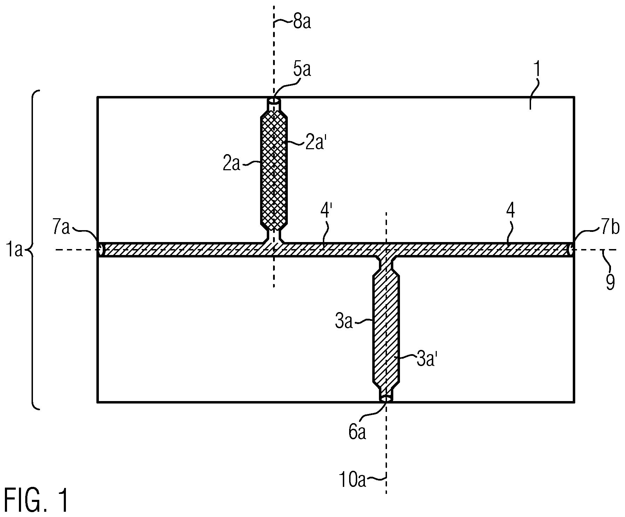

In Figuren 1 bis 4 sind erste bis vierte Ausführungsformen eines erfindungsgemäßen Substrats 1 gezeigt. In jeder der hier gezeigten Ausführungsformen ist in dem Substrat 1 ein Fluidkanalsystem 1a ausgebildet, umfassend einen ersten Kultivierungskanal 2a, einen Speicherkanal 3a und einen Überführungskanal 4. Der erste Kultivierungskanal weist einen Kultivierungszugang 5a auf. Der Speicherkanal weist einen Speicherzugang 6a auf und der Überführungskanal weist einen Überführungszugang 7a auf. Der Überführungskanal kann weiterhin optional einen zweiten Überführungszugang 7b aufweisen.

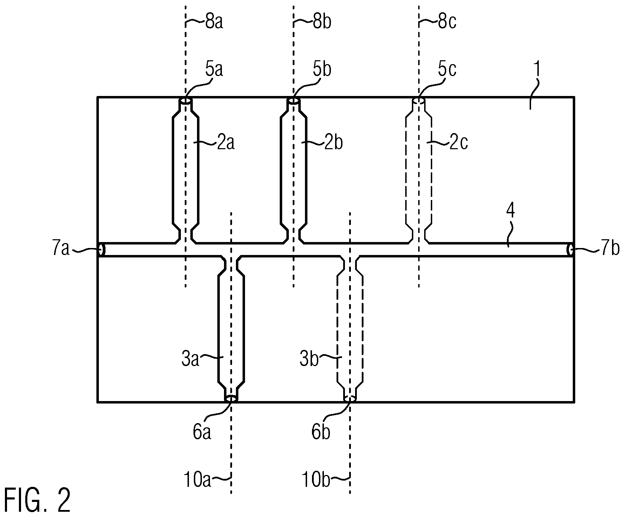

In Figur 1 ist beispielhaft angedeutet, dass der erste Kultivierungskanal eine Beschichtung 2a' aufweisen kann. Dabei kann es sich insbesondere um eine zelladhärierende Beschichtung oder eine hydrophile Beschichtung handeln. Außerdem ist beispielhaft angedeutet, dass der Überführungskanal und der Speicherkanal eine Beschichtung 4' bzw. 3a' aufweisen. Dabei kann es sich jeweils um eine zellabweisende oder eine hydrophobe Beschichtung handeln. Die Beschichtungen sind in den Figuren 2 bis 4 nicht gezeigt, können in den entsprechenden Ausführungsformen jedoch ebenfalls vorgesehen sein.

In den in Figuren 2 bis 4 gezeigten Ausführungsformen umfasst das Fluidkanalsystem zudem einen oder mehrere weitere Kultivierungskanäle, beispielsweise einen zweiten Kultivierungskanal 2b und einen dritten Kultivierungskanal 2c, und einen oder mehrere weitere Speicherkanäle, beispielsweise einen zweiten Speicherkanal 3b und einen dritten Speicherkanal 3c. Der bzw. die zusätzlichen Kultivierungskanäle weisen jeweils einen eigenen Kultivierungszugang 5b bzw. 5c auf. Der bzw. die zusätzlichen Speicherkanäle weisen jeweils einen eigenen Speicherzugang 6b bzw.6c auf.

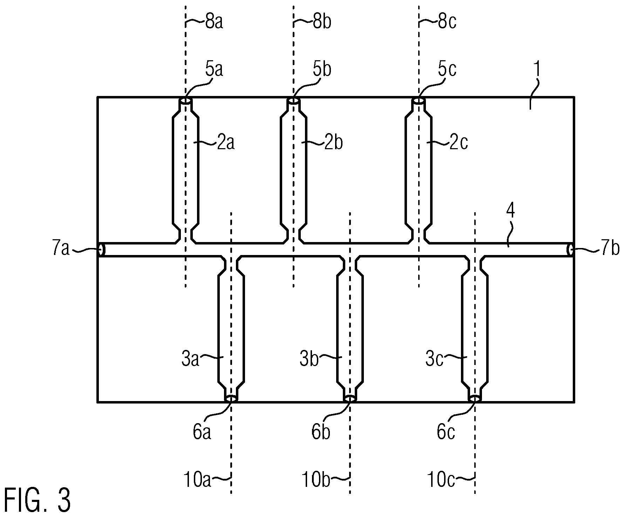

In Figur 2 sind die Kanäle 2b, 2c und 3b gestrichelt eingetragen. Dies verdeutlicht, dass eine beliebige Anzahl an zusätzlichen Kanälen vorgesehen sein kann, wobei insbesondere die Anzahl der Speicherkanäle und die Anzahl der Kultivierungskanäle nicht gleich sein müssen. In Figur 3 ist hingegen eine Ausführungsform gezeigt, in der die Anzahl der Speicherkanäle gleich der Anzahl der Kultivierungskanäle ist.

Wie in den Figuren 1 bis 4 gezeigt, ist jeder der Kultivierungskanäle fluidisch mit dem Überführungskanal verbunden. In diesem Beispiel ist jeder der Kultivierungskanäle zum Überführungskanal hin verengt, das heißt, der Durchmesser der Mündung des jeweiligen Kanals ist kleiner als der Durchmesser in anderen Bereichen des Kanals, was jedoch optional ist. Außerdem ist jeder der Speicherkanäle ist fluidisch mit dem Überführungskanal verbunden. Die Längsachsen 8a bis 8c der Kultivierungskanäle sind im Wesentlichen senkrecht zu der Längsachse 9 des Überführungskanals angeordnet, was jedoch nicht zwingend erforderlich ist.

In den Figuren 1 bis 3 ist jeder der Speicherkanäle zum Überführungskanal hin verengt was jedoch optional ist. Zudem sind hier die Längsachsen 10a bis 10c der Speicherkanäle entlang des Überführungskanals versetzt zu den Längsachsen 8a bis 8c der Kultivierungskanäle angeordnet, hier beispielhaft ebenfalls im Wesentlichen senkrecht zur Längsachse des Überführungskanals.

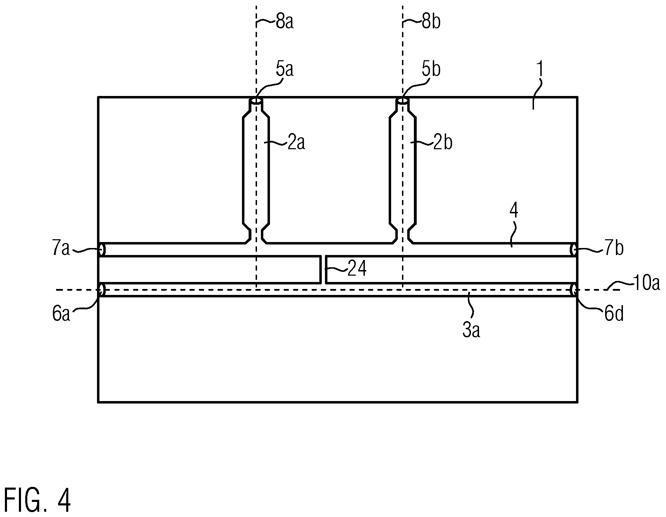

In Figur 4 ist die Längsachse 10a des Speicherkanals 3a im Wesentlichen parallel zu der Längsachse des Überführungskanals angeordnet. Das Substrat weist eine Fluidverbindung 24 zwischen dem Speicherkanal und dem Überführungskanal auf. Die Fluidverbindung hat einen Durchmesser, der kleiner ist als der Durchmesser des Speicherkanals, und ihre Längsachse ist in diesem Beispiel im Wesentlichen senkrecht zur Längsachse des Überführungskanals und entlang des Überführungskanals versetzt zu den Längsachsen der Kultivierungskanäle angeordnet. Der Speicherkanal 3a in Figur 4 kann zusätzlich zu dem Speicherzugang 6a einen weiteren Speicherzugang 6d aufweisen.

In der ersten bis vierten Ausführungsform sind beispielhaft alle Kanäle in einer Ebene angeordnet. Insbesondere sind bei der bestimmungsgemäßen Verwendung des Substrats ihre Längsachsen horizontal angeordnet.

Das Substrat kann transparent, insbesondere aus einem transparenten Kunststoffmaterial, sein ausgebildet. Das Substrat kann insbesondere derart ausgebildet sein, dass die darin befindlichen Zellen mikroskopisch untersucht werden können.

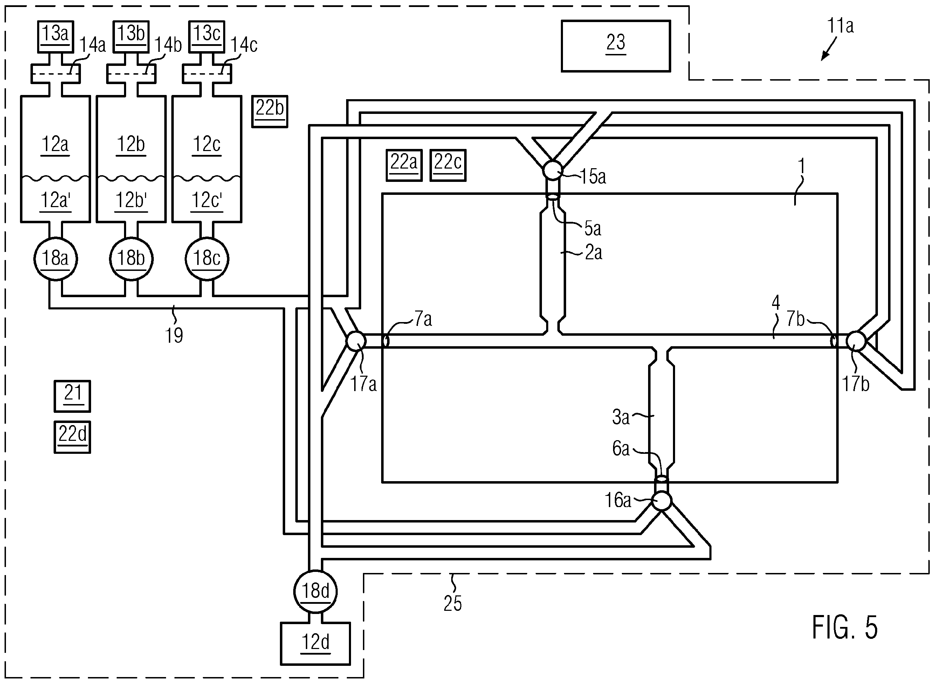

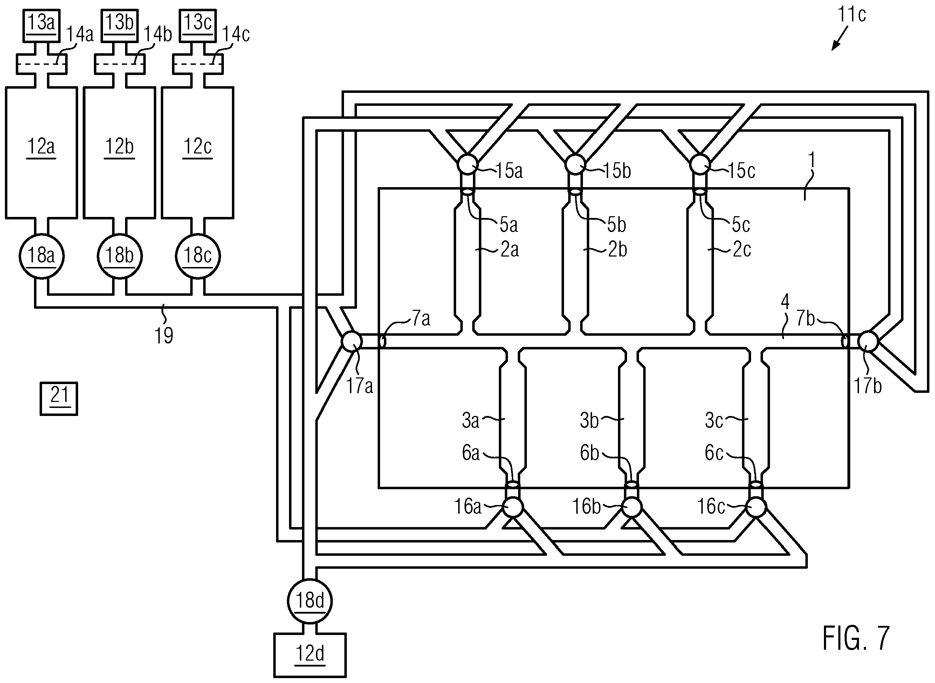

In den Figuren 5 bis 8 sind schematisch und nicht maßstabsgetreu fünfte bis achte Ausführungsformen eines Systems 11a bis 11d zum Kultivieren von Zellen gezeigt, hier beispielhaft umfassend die in Figuren 1 bis 4 gezeigten Substrate. Dies ist jedoch optional und andere erfindungsgemäße Substrate können in jedem der Systeme verwendet werden. Insbesondere kann die Form, Ausrichtung, Anzahl, Verbindung untereinander und/oder Beschichtung der Kanäle in dem Fluidkanalsystem anders sein als im Zusammenhang mit Figuren 1 bis 4 beschrieben.

In Figuren 5 bis 8 sind jeweils beispielhaft drei Speicherbehälter für Prozessflüssigkeiten, beispielsweise ein erster Speicherbehälter 12a für Ausgangszellsuspension 12a', ein zweiter Speicherbehälter 12b für Zellkulturmedium 12b' und ein dritter Speicherbehälter 12c für eine Flüssigkeit 12c' zum Ablösen der Zellen. Es versteht sich, dass weitere Speicherbehälter für Prozessflüssigkeiten vorgesehen sein können. Weiterhin kann das System, wie in den Figuren gezeigt, optional einen Abfallbehälter 12d umfassen. An jeden der Speicherbehälter 12a bis 12c kann jeweils eine Pumpe 13a bis 13c angeschlossen sein. Dabei kann jeweils zwischen Pumpe und Speicherbehälter optional ein Filter 14a bis 14c, beispielsweise ein Sterilfilter, angeordnet sein. Je nach Prozess kann auch eine andere Anzahl an Speicherbehältern vorgesehen sein. Gegebenenfalls kann auch für mehrere Speicherbehälter nur eine Pumpe vorgesehen sein. Das System kann dann beispielsweise ein Ventil umfassen, das so ausgebildet ist, dass damit eingestellt werden kann, an welchen der Speicherbehälter Druck angelegt wird.

An jedem der Zugänge kann mindestens ein Ventil angeschlossen sein. In den Figuren ist der Kultivierungszugang 5a mit einem ersten Ventil 15a verbunden, der Speicherzugang 6a ist mit einem zweiten Ventil 16a verbunden und der Überführungszugang 7a ist mit einem dritten Ventil 17a verbunden. Sofern vorhanden, können die weiteren Kultivierungszugänge 5b und 5c jeweils mit einem vierten Ventil 15b und 15c, der weitere Überführungszugang 7b mit einem fünften Ventil 17b, die weiteren Speicherzugänge 6b und 6c jeweils mit einem sechsten Ventil 16b und 16c bzw. der Zugang 6d mit einem weiteren Ventil 16d verbunden sein.

Wie in Figuren 5 bis 8 gezeigt, können die Speicherbehälter 12a, 12b und 12c jeweils über ein Ventil 18a, 18b, 18c mit einer Leitung 19 verbunden sein. Die Leitung kann mit dem Kultivierungszugang 5a des ersten Kultivierungskanals über das Ventil 15a, mit dem Überführungszugang 7a des Überführungskanals über das Ventil 17a und optional mit dem Speicherzugang 6a des Speicherkanals über das Ventil 16a verbunden sein. Auch einer oder mehrere der übrigen Zugänge 5b, 5c, 6b, 6c, 6d und/oder 7b können jeweils über ein Ventil 15b, 15c, 16b, 16c, 16d bzw. 17b mit der Leitung verbunden sein. Jedem der Zugänge, der mit der Leitung verbunden ist kann durch geeignete Ventilstellung der Ventile 18a bis 18c und des dem jeweiligen Zugang zugeordneten Ventils eine der Flüssigkeiten in den Flüssigkeitsbehältern 12 a bis 12c zugeführt werden.

Jeder der Zugänge kann, alternativ oder zusätzlich zu einer Verbindung mit der Leitung, über das entsprechende Ventil mit dem Abfallbehälter 12d verbunden sein, optional über einen Durchflussmesser 18d. Anhand der Messwerte des Durchflussmessers kann eindeutig der Durchfluss im System bestimmt werden, wenn es sich, wie hier gezeigt, um ein geschlossenes System handelt. Die Messwerte des Durchflussmessers 18d können dann als Steuer- und/oder Regelgröße, insbesondere für das Betätigen der Ventile, verwendet wird.

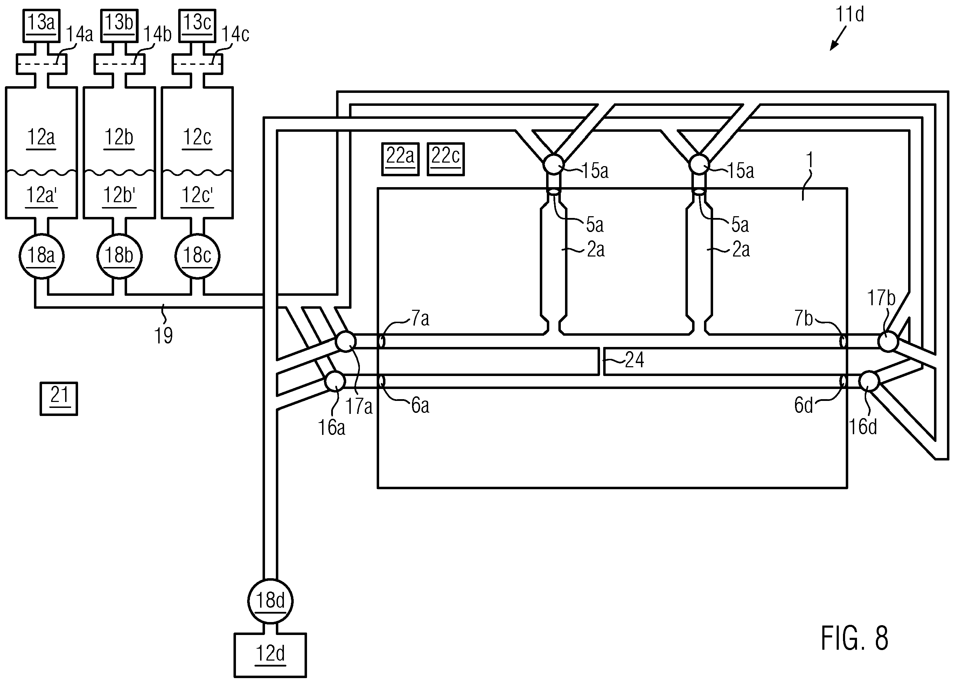

In der in Figur 8 gezeigten Ausführungsform weist der Speicherkanal 3a zwei Zugänge 6a und 6d auf. Hier sind beide Zugänge über Ventile mit der Leitung und dem Abfallbehälter verbunden. Alternativ kann einer der Zugänge, beispielsweise Zugang 6a über das Ventil 16a, ausschließlich mit der Leitung 19 und der andere Zugang, beispielsweise Zugang 6d über das Ventil 16d, ausschließlich mit dem Abfallbehälter 12d verbunden sein.

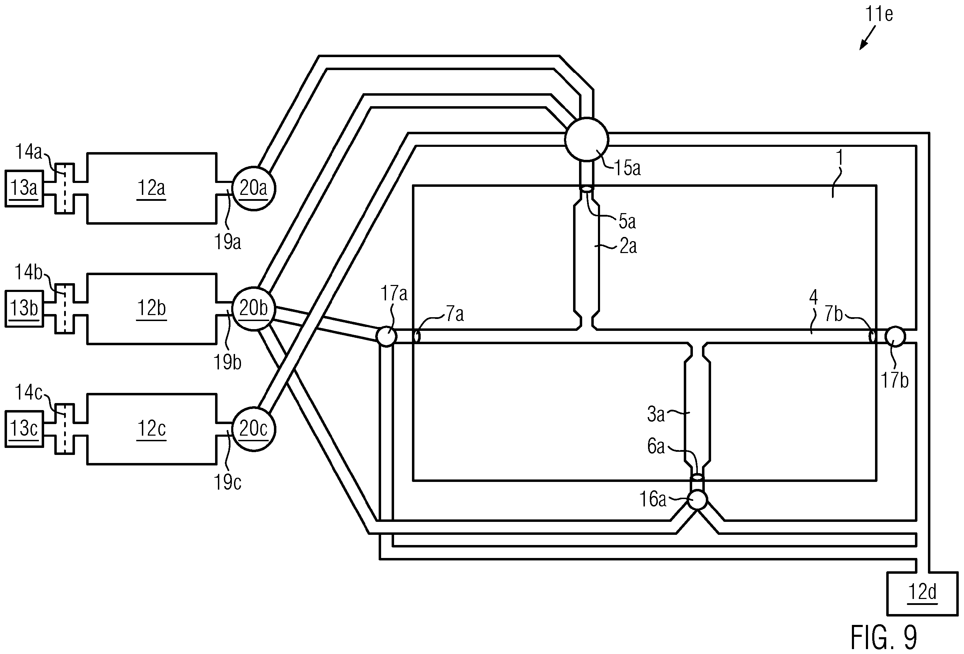

In Figur 9 ist ein alternatives System 11e gezeigt. Das System umfasst ebenfalls vier Flüssigkeitsbehälter 12a bis 12d, beispielsweise die oben beschriebenen Speicherbehälter und Abfallbehälter, drei Pumpen 13a bis 13c und Folter 14a bis 14c. Weiterhin zeigt es Ventile 15a, 16a, 17a und 17b, die jeweils mit den Zugängen 5a, 6a, 7a, 7b verbunden sind. Beispielhaft ist das System mit dem Substrat der ersten Ausführungsform gezeigt, aber jedes erfindungsgemäße Substrat kann stattdessen verwendet werden. Hier sind die drei Flüssigkeitsbehälter nicht mit einer gemeinsamen Leitung 19 verbunden. Jeder Der Flüssigkeitsbehälter weist eine eigene Leitung 19a bis 19c auf und die Flüssigkeit kann jeweils über ein Ventil 20a bis 20c einem der Zugänge über das mit dem jeweiligen Zugang verbundene Ventil zugeführt werden.

In der hier gezeigten Ausführungsform kann Ausgangszellsuspension aus dem Behälter 12a über das Ventil 20a und das Ventil 15a dem ersten Kultivierungskanal 2a zugeführt werden. Flüssigkeit aus dem Behälter 12b kann über das Mehrwegeventil 20b den Zugängen 15a, 16a oder 17a zugeführt werden. Flüssigkeit aus dem Behälter 12c, beispielsweise Flüssigkeit zum Ablösen von Zellen, kann über das Ventil 20c dem ersten Kultivierungskanal zugeführt werden. Außerdem kann Flüssigkeit aus dem ersten Kultivierungskanal 2a durch das Ventil 15a dem Abfallbehälter 12d zugeführt werden, Flüssigkeit aus dem Überführungskanal 4 kann durch das Ventil 17b dem Abfallbehälter zugeführt werden und Flüssigkeit aus dem Speicherkanal 3a kann über das Ventil 16a dem Abfallbehälter zugeführt werden.

In jeder der Figuren 5 bis 9 ist jeweils eine optional vorgesehene Steuereinrichtung 21 gezeigt.

In Figur 5 sind optional vorgesehene Sensoren 22a bis 22d gezeigt. Sensor 22a ist beispielsweise zum Messen der Luftfeuchtigkeit im Bereich des Substrats ausgebildet und angeordnet. Sensor 22b ist zum Messen der Temperatur im Bereich der Flüssigkeitsbehälter ausgebildet und angeordnet. Sensor 22c ist zum Messen der Temperatur im Bereich des Substrats ausgebildet und angeordnet. Sensor 22d kann ein Sensor zum Messen des Sauerstoff- oder CO₂-Anteils der Luft im System sein. Insbesondere können einer oder mehrere der Sensoren zusammen mit dem Substrat und/oder den Flüssigkeitsbehältern innerhalb eines Gehäuses 25 angeordnet sein.

Weiterhin ist schematisch eine optional vorgesehene Einrichtung zur optischen Untersuchung 23 angedeutet. Dabei kann es sich um eine Mikroskopiereinrichtung handeln. Das Substrat kann in Form eines Mikroskopieträgers ausgebildet sein. Die Einrichtung zur optischen Untersuchung kann zumindest teilweise innerhalb des Gehäuses angeordnet sein, was eine Überwachung ohne Eingriff in das System ermöglicht. Die Einrichtung zur optischen Untersuchung kann alternativ außerhalb des Gehäuses angeordnet und das Gehäuse abnehmbar sein.

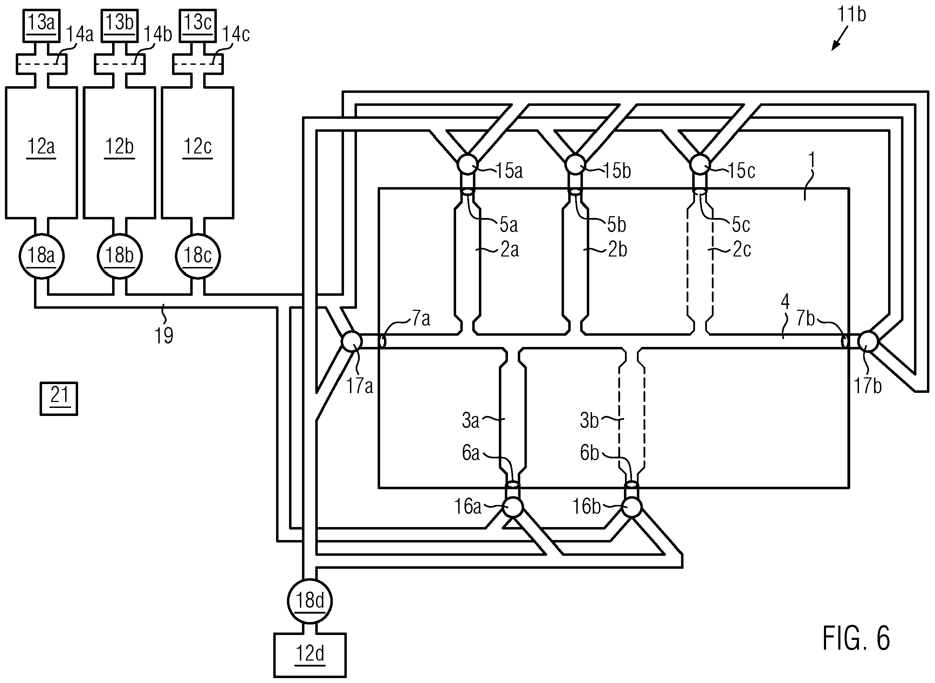

Die Sensoren, Einrichtung zur optischen Untersuchung und das Gehäuse sind in den Figuren 6 bis 9 nicht dargestellt, aber auch die dort gezeigten Systeme können einen oder mehrere dieser Sensoren, eine Einrichtung zur optischen Untersuchung und/oder ein Gehäuse umfassen.

Im Folgenden wird ein Verfahren zum Kultivieren von Zellen, hier beispielhaft anhand des Systems der sechsten Ausführungsform, wie in Figur 6 gezeigt, erläutert. Es kann analog auch mit anderen, insbesondere den oben beschriebenen, Systemen durchgeführt werden. Im Folgenden werden an entsprechender Stelle beispielhaft mögliche Abwandlungen erläutert, die für ein anders ausgebildetes System geeignet sind.

In dem Flüssigkeitsbehälter 12a befindet sich in diesem Beispiel eine Ausgangszellsuspension, eine Flüssigkeit, in der die auszusäenden Zellen suspendiert sind. In dem Flüssigkeitsbehälter 12b befindet sich ein Zellkulturmedium. In dem Flüssigkeitsbehälter 12c befindet sich eine Flüssigkeit zum Ablösen von Zellen, beispielsweise Trypsin. Sofern eine solche Flüssigkeit für den entsprechenden Anwendungsfall nicht erforderlich ist, versteht es sich, dass auf diesen Speicherbehälter verzichtet werden kann.

Im ersten Schritt wird unter Verwendung der Pumpe 13a Flüssigkeit aus dem Speicherbehälter 12a in den ersten Kultivierungskanal transportiert. Dazu können die Ventile 15a, 17a und 18a betätigt werden. Alle anderen Ventile sind in diesem Beispiel geschlossen. Das Öffnen des Ventils 18a ermöglicht, Ausgangszellsuspension aus dem Speicherbehälter 12a in die Leitung 19 gelangt.

Je nachdem, ob das Ventil 15a oder 17a zu der Leitung hin offen ist, wird die Ausgangszellsuspension über den Zugang 5a bzw. 7a in den ersten Kultivierungskanal transportiert, sofern das jeweils andere Ventil zu dem Abfallbehälter 12d hin offen ist. Die Ausgangszellsuspension verbleibt im Kultivierungskanal oder strömt langsam durch den ersten Kultivierungskanal. Die Zellen setzen sich im ersten Kultivierungskanal ab und können an den Wänden des Kanals adhärieren. Die Flüssigkeit der Ausgangszellsuspension kann dann optional aus dem ersten Kultivierungskanal in den Abfallbehälter transportiert werden oder zunächst im Kultivierungskanal verbleiben.

Im ersten Kultivierungskanal beginnen sich die Zellen zu vermehren. Optional kann, beispielsweise über den gleichen Weg wie die Ausgangszellsuspension, eine Nährflüssigkeit in den ersten Kultivierungskanal transportiert und anschließend, wenn die Nährstoffe verbraucht sind, daraus abgeführt werden.

In einem optionalen zweiten Schritt wird, wenn sich die Zellen vermehrt und eine erste Zellkultur gebildet haben, unter Verwendung der Pumpe 13c die Flüssigkeit zum Ablösen der Zellen aus dem Speicherbehälter 12c in die erste Kultivierungskammer transportiert. Dazu werden die Ventile 15a, 17a und 18c betätigt. Alle anderen Ventile sind in diesem Beispiel geschlossen. Das Öffnen des Ventils 18c ermöglicht, dass die Flüssigkeit aus dem Speicherbehälter 12c in die Leitung 19 gelangt. Von dort gelangt die Flüssigkeit wie zuvor die Ausgangszellsuspension in die erste Kultivierungskammer. Der zweite Schritt kann entfallen, wenn die Zellen nicht oder nicht sehr stark an den Wänden der Kultivierungskammer anwachsen. Insbesondere kann bei einem nicht starken Anwachsen das Ablösen unter Umständen rein mechanisch, beispielsweise durch Scherspannung, erfolgen.

In einem dritten Schritt werden die Zellen aus dem ersten Kultivierungskanal über den Überführungskanal in den ersten Speicherkanal überführt. Dazu wird das Ventil 18b geöffnet, so dass Zellkulturmedium aus dem Flüssigkeitsbehälter 12b in die Leitung 19 gelangt. Das Zellkulturmedium wird unter Verwendung der Pumpe 13b durch den Kultivierungszugang 5a in den ersten Kultivierungskanal eingeleitet und durch den Überführungskanal in den Speicherkanal transportiert. Dabei werden die Zellen in den Speicherkanal überführt. In diesem Schritt wird das Ventil 15a zur Leitung hin geöffnet. Außerdem wird das Ventil 16a geöffnet, beispielsweise zu einem Abfallbehälter hin.

Das erfindungsgemäße Verfahren zum Kultivieren von Zellen kann bereits nach der Überführung der Zellen in den Speicherkanal enden. Die Zellen können im Speicherkanal zwischengespeichert und/oder untersucht werden.

Optional kann das Verfahren weiterhin einen oder mehrere der folgenden Schritte umfassen.

Beispielsweise kann in einem optionalen vierten Schritt eine Konzentration der Zellsuspension erfolgen, beispielsweise im Speicherkanal. Dazu wird die Eigenschaft der Zellen genutzt, dass sie auf den Boden absinken. Durch Einstellen einer ausreichend niedrigen Fließgeschwindigkeit und optional unterstützt durch eine Strukturierung des Bodens des Speicherkanals verbleiben die Zellen in dem Speicherkanal, während ein Teil des Zellkulturmediums aus dem Speicherkanal weiter transportiert wird, so dass die Zellkonzentration erhöht wird.

Alternativ oder zusätzlich kann in einem optionalen fünften Schritt die Zellsuspension im Speicherkanal homogenisiert werden, beispielsweise indem sie gemischt wird. Optional kann nach der Homogenisierung eine optische Untersuchung der Zellen erfolgen, die nun gleichmäßiger im Speicherkanal angeordnet sind. Wenn aufgrund der Homogenisierung eine homogene Zellverteilung vorliegt, genügt eine optische Untersuchung der Zellen an wenigen Positionen im Substrat, um über die gesamte Zellkultur repräsentative Aussagen zu treffen.

Alternativ oder zusätzlich kann in einem optionalen sechsten Schritt eine Verdünnung der im Speicherkanal oder im Überführungskanal befindlichen Zellsuspension erfolgen. Dazu kann das Ventil 18c geöffnet werden, so dass Zellkulturmedium in die Leitung gelangt, und mittels der Pumpe 13c in den Speicherkanal 3a transportiert werden, insbesondere durch den Zugang 6a über das Ventil 16a. Zeitgleich ist ein anderes Ventil, beispielsweise das Ventil 15a oder, sofern vorhanden, das Ventil 15b, 17a oder 17b, beispielsweise zum Abfallbehälter hin offen.

Wenn im Anschluss an diese Schritte keine weitere Kultivierung der Zellen im Substrat erfolgen soll, kann ein Substrat ohne weitere Kultivierungskanäle verwendet werden, beispielsweise wie in Figuren 1 und 5 gezeigt.

Wenn das Verfahren weitere Kultivierungsschritte umfasst, erfolgt als siebter Schritt eine Überführung der Zellen durch den Überführungskanal in den zweiten Kultivierungskanal 2b. Dazu kann das Ventil 18b geöffnet werden, so dass Zellkulturmedium in die Leitung gelangt, und mittels der Pumpe 13b in den Speicherkanal 6a transportiert werden, insbesondere wie hier gezeigt durch den Zugang 6 über das Ventil 16a. Außerdem wird das Ventil 15b, beispielsweise zum Abfallbehälter hin, geöffnet. Das Zellkulturmedium wird dann zusammen mit den Zellen aus dem Speicherkanal über den Überführungskanal in den zweiten Kultivierungskanal transportiert. Dort können die Zellen sich vermehren und so eine zweite Zellkultur gezüchtet werden.

Die oben beschriebenen Schritte können für weiteren Kultivierungskammern wiederholt werden.

Dabei können die Zellen jeder der Zellkulturen jeweils in dem ersten Speicherkanal zwischengelagert werden oder jeweils in einen separaten dem jeweiligen Kultivierungskanal zugeordneten Speicherkanal, beispielsweise Speicherkanäle 3b oder 3c, transportiert und dort gelagert und optional weiterverarbeitet und/oder untersucht werden.

Es ist auch denkbar, dass in einem Substrat mehrere Kultivierungskanäle vorgesehen sind, denen Ausgangszellsuspension zugeführt wird. Mit anderen Worten können mehrere erste Kultivierungskanäle vorgesehen sein. So können im ersten Kultivierungsschritt bereits mehrere erste Zellkulturen gezüchtet werden. Mit anderen Worten können parallel zueinander mehrere erste Zellkulturen gezüchtet werden. So können innerhalb kurzer Zeit viele Zellen gezüchtet werden. Wenn stattdessen die selbe Anzahl an Kultivierungskanälen sequentiell genutzt wird, kann das Verfahren über einen längeren Zeitraum automatisiert durchgeführt werden ohne Kanäle doppelt zu verwenden.

Wenn weitere Kultivierungsschritte jeweils in weiteren Kultivierungskanälen erfolgen, kann eine gleichbleibende Oberflächenqualität für jeden der Kultivierungsschritte leicht sichergestellt werden. Das Verfahren kann dann ohne Aufbereitung oder Reinigung des ersten Kultivierungskanals erfolgen. Ein zweiter Kultivierungsschritt im ersten Kultivierungskanal statt oder zusätzlich zu einem Kultivierungsschritt in einem anderen Kultivierungskanal ist jedoch ebenfalls denkbar. In diesem Fall würde eine Rückführung aus dem Speicherkanal in den ersten Kultivierungskanal durch den Überführungskanal erfolgen, wobei insbesondere ein Reinigungsschritt zum Reinigen des Kultivierungskanals zwischen den Kultivierungsschritten durchgeführt werden kann. Das hat den Vorteil, dass ein kompakteres Substrat verwendet werden kann.

Ein detailliertes Verfahren ist im Folgenden beispielhaft anhand eines Systems mit mindestens zwei Kultivierungskanälen, beispielsweise ein System wie in Figur 6, 7 oder 8 gezeigt, beschrieben. Alle Ventile, die nicht als offen beschrieben sind, sind im jeweiligen Schritt geschlossen. Das Verfahren kann folgende Schritte umfassen.

Zellaussaat im ersten Kultivierungskanal 2a: Ventil 18a ist zur Leitung hin offen, Ventil 15a ist zum Abfallbehälter hin offen, Ventil 17a ist zur Leitung hin offen. Der angelegte Druck kann beispielsweise 20 mbar betragen. Die Ausgangszellsuspension fließt aus dem Speicherbehälter 12a durch die Leitung und den Überführungskanal in den Kultivierungskanal 2a. Dort sinken die Zellen auf den Boden ab und adhärieren. Zellteilung beginnt. Alternativ dazu können Ventil 15a zur Leitung hin offen und Ventil 17a zum Abfallbehälter hin offen sein. Die Zellen werden dann direkt (also nicht über den Überführungskanal) dem Kultivierungskanal zugeführt.

Wegwaschen nicht adhärenter und toter Zellen, beispielsweise 5 min bis 60 min, insbesondere 20 min, nach der Zellaussaat: Ventil 18b ist zur Leitung hin offen, Ventil 15a ist zum Abfallbehälter hin offen, Ventil 17a ist zur Leitung hin offen. Der angelegte Druck kann beispielsweise 50 mbar betragen. Zellsuspension wird über den Überführungskanal durch den Kultivierungskanal gespült, um nicht adhärierende und tote Zellen wegzuspülen. Alternativ können Ventil 15a zur Leitung hin offen und Ventil 17a zum Abfallbehälter hin offen sein. Die Zellen werden dann direkt (also nicht über den Überführungskanal) aus dem Kultivierungskanal aus dem Substrat abtransportiert.

Trypsinierung, beispielsweise wenn die Zellkultur eine vorgegebene Zelldichte aufweist, beispielsweise nach 48 Stunden: Ventil 18c ist zur Leitung hin offen, Ventil 15a ist zum Abfallbehälter hin offen, Ventil 17a ist zur Leitung hin offen. Der angelegte Druck kann beispielsweise 20 mbar betragen. Trypsinhaltige Lösung fließt aus dem Speicherbehälter12c durch den Überführungskanal in den Kultivierungskanal 2a, verbleibt dort und löst Zellen ab. Die Lösung kann so lange einwirken, bis eine vorgegebene Menge an Zellen von der Kanaloberfläche gelöst sind, beispielsweise etwa 5 Minuten.

Zellüberführung und Konzentrieren: Ventil 18b ist zur Leitung hin offen, Ventil 15a ist zur Leitung hin offen, Ventil 16a ist zum Abfallbehälter hin offen. Der angelegte Druck kann beispielsweise 100 mbar betragen. Zellkulturmedium aus dem Speicherbehälter 12b wird durch den ersten Kultivierungskanal, den Überführungskanal und den Speicherkanal hindurch gepumpt. Beispielsweise kann das zugeführte Flüssigkeitsvolumen etwa das 1,2-fache Volumen des Kultivierungskanals sein. Dabei werden Zellen durch den Überführungskanal in den Speicherkanal transportiert. Zellen, die noch an der Oberfläche des Kultivierungskanals anhaften, können durch die Scherkraft abgelöst und ebenfalls überführt werden. Im Speicherkanal können die Zellen zurückgehalten werden, beispielsweise durch Barrieren, die in Form einer Strukturierung des Bodens des Speicherkanals ausgebildet sein können, oder durch eine Membran am Zugang des Speicherkanals. Zellkulturmedium wird abtransportiert und Zellen verbleiben im Speicherkanal.

Verdünnung: Ventil 18b ist zur Leitung hin offen, Ventil 16a ist zur Leitung hin offen, Ventil 17b ist zum Abfallbehälter hin offen. Der angelegte Druck kann beispielsweise 20 mbar betragen. Zellkulturmedium aus dem Speicherbehälter 12b wird durch den Speicherkanal und den Überführungskanal aus dem Substrat heraus transportiert. Dabei werden Zellen kontrolliert aus dem Speicherkanal transportiert. Abhängig von der Dauer bzw. Menge an Flüssigkeit und Fließgeschwindigkeit kann die Menge an verbleibenden Zellen eingestellt werden. Beispielsweise kann das zugeführte Flüssigkeitsvolumen etwa das 2-fache Volumen des Speicherkanals sein.

Optionales Durchmischen von Flüssigkeit mit den Zellen im Speicherkanal, um eine homogenere Zellverteilung zu erhalten: Dazu kann die Flüssigkeit durch Anlegen von Über- und Unterdruck hin und her bewegt werden. Dieser Schritt kann vor und/oder nach der Verdünnung erfolgen.

Überführung in den und Zellaussaat in dem zweiten Kultivierungskanal 2b: Ventil 18b ist zur Leitung hin offen, Ventil 15b ist zum Abfallbehälter hin offen, Ventil 16a ist zur Leitung hin offen. Der angelegte Druck kann beispielsweise 20 mbar betragen. Zellkulturmedium aus dem Speicherbehälter 12b überführt Zellen aus dem Speicherkanal 6 übe den Überführungskanal in den zweiten Kultivierungskanal 2b. Dort sinken die Zellen auf den Boden ab und adhärieren. Zellteilung beginnt. Optional kann zusätzlich Ventil 17a zur Leitung hin offen sein und Zellkulturmedium kann durch den Überführungszugang 7a in das Substrat eingeleitet und während der Überführung der Zellen im Überführungskanal mit den aus dem Speicherkanal transportierten Zellen zusammengeführt werden, beispielsweise um eine Verdünnung (alternativ oder zusätzlich zur oben genannten Verdünnung im Speicherkanal) durchzuführen.

Alternativ zur Zellaussaat im zweiten Kultivierungskanal: Entnahme der Zellen aus dem Substrat.

Die obige Abfolge von Schritten kann wiederholt werden, wobei bei jeder Wiederholung ein weiterer oder derselbe Speicherkanal verwendet werden kann.

Es versteht sich, dass in den zuvor beschriebenen Ausführungsbeispielen genannte Merkmale nicht auf diese speziellen Kombinationen beschränkt sind und auch in beliebigen anderen Kombinationen möglich sind.

## Patentansprüche

1. Verfahren zum Kultivieren von Zellen in einem Substrat (1),
wobei in dem Substrat (1), ein Fluidkanalsystem (1a) ausgebildet ist, das einen Kultivierungskanal (2a, 2b, 2c) mit einem Kultivierungszugang (5a, 5b, 5c) nach außen, einen Speicherkanal (3a, 3b, 3c) mit einem Speicherzugang (6a, 6b, 6c) nach außen und einen Überführungskanal (4) mit einem Überführungszugang (7a, 7b) nach außen umfasst,
wobei der Kultivierungskanal (2a, 2b, 2c) und der Speicherkanal (3a, 3b, 3c) fluidisch über den Überführungskanal (4) miteinander verbunden sind, und
wobei das Verfahren umfasst:
Zuführen einer Ausgangszellsuspension (12a') durch den Kultivierungszugang (5a, 5b, 5c) in den Kultivierungskanal (2a, 2b, 2c) oder durch den Überführungszugang (7a, 7b) über den Überführungskanal (4) in den Kultivierungskanal;
Züchten einer Zellkultur in dem Kultivierungskanal;
Überführen von Zellen der Zellkultur aus dem Kultivierungskanal (2a, 2b, 2c) in den Speicherkanal (3a, 3b, 3c) über den Überführungskanal (4), wobei das Überführen durch Zuführen einer Überführungsflüssigkeit durch den Kultivierungszugang (5a, 5b, 5c) und Transport der Überführungsflüssigkeit durch den Kultivierungskanal (2a, 2b, 2c) und den Überführungskanal (4) in den Speicherkanal (3a, 3b, 3c) erfolgt; und
Speichern und/oder Konzentrieren und/oder Homogenisieren und/oder Verdünnen einer die überführten Zellen enthaltenden Zellsuspension in dem Speicherkanal (3a, 3b, 3c).

2. Verfahren nach Anspruch 1, wobei alle Verfahrensschritte, bei denen Flüssigkeiten in dem Fluidkanalsystem (1a) bewegt werden, mittels ausschließlich außerhalb des Substrats (1) angeordneten aktiven Komponenten (13a-13c, 15a-15c, 16a-16d, 17a, 17b, 18a-18c), insbesondere Pumpen und/oder Ventilen, erfolgen, insbesondere automatisch durch Ansteuern der aktiven Komponenten (13a-13c, 15a-15c, 16a-16d, 17a, 17b, 18a-18c).

3. Verfahren nach Anspruch 2,
wobei durch das Betätigen der aktiven Komponenten (13a-13c, 15a-15c, 16a-16d, 17a, 17b, 18a-18c), insbesondere automatisch durch Ansteuern der aktiven Komponenten (13a-13c, 15a-15c, 16a-16d, 17a, 17b, 18a-18c), eingestellt wird,
welche Flüssigkeit dem Fluidkanalsystem (1a) zugeführt wird, und/oder
eine Reihenfolge von Flüssigkeiten, die dem Fluidkanalsystem (1a) zugeführt werden, und/oder
durch welchen Zugang Flüssigkeit dem Fluidkanalsystem (1a) zugeführt wird, und/oder
durch welchen Zugang Flüssigkeit aus dem Fluidkanalsystem (1a) entnommen wird, und/oder
der Transportweg von Flüssigkeit im Fluidkanalsystem (1a), und/oder
die Fließgeschwindigkeit von Flüssigkeit im Fluidkanalsystem (1a), und/oder
wann Flüssigkeit zugeführt und/oder weitertransportiert und/oder abgeführt wird,
wobei das Einstellen erfolgt insbesondere, indem
Verbindungen zwischen einem Flüssigkeitsbehälter (12a-12d), in dem die jeweilige Flüssigkeit gelagert wird, und dem Fluidkanalsystem (1a) hergestellt und/oder unterbrochen werden, beispielsweise durch, insbesondere automatisches, Öffnen und/oder Schließen von Ventilen und/oder Schalten von Mehrwegeventilen, und/oder
ein zum Transport der jeweiligen Flüssigkeit in das Fluidkanalsystem (1a) und/oder in dem Fluidkanalsystem (1a) geeigneter Druck angelegt wird, beispielsweise durch, insbesondere automatisches, Betätigen von Pumpen.

4. Verfahren nach einem der vorangegangenen Ansprüche, wobei in dem Substrat (1) keine aktiven Komponenten (13a-13c, 15a-15c, 16a-16d, 17a, 17b, 18a-18c) und/oder keine Messeinrichtungen (22a-22d, 23) und/oder keine Steuereinrichtungen (20) angeordnet sind.

5. Verfahren nach einem der vorangegangenen Ansprüche, wobei die Zellen der Zellkultur enzymatisch und/oder mittels beim Bewegen einer Flüssigkeit in dem jeweiligen Kultivierungskanal (2a, 2b, 2c) entstehende Scherkräfte von den Wänden des Kultivierungskanals (2a, 2b, 2c) abgelöst werden, bevor sie über den Überführungskanal (4) in den Speicherkanal (3a, 3b, 3c) überführt werden.

6. Verfahren nach einem der vorangegangenen Ansprüche umfassend, während des Züchtens und/oder während des Ablösens und/oder während die Zellen im Speicherkanal (3a, 3b, 3c) angeordnet sind, optische Untersuchung der Zellen.

7. Verfahren nach einem der vorangegangenen Ansprüche,
wobei der Kultivierungskanal (2a, 2b, 2c) ein erster Kultivierungskanal (2a, 2b, 2c) ist und die Überführungsflüssigkeit eine erste Überführungsflüssigkeit ist, und
wobei das Fluidkanalsystem (1a) einen zweiten Kultivierungskanal (2a, 2b, 2c) umfasst, der fluidisch mit dem Überführungskanal (4) verbunden ist, und
wobei das Verfahren ein Überführen der Zellen aus dem Speicherkanal (3a, 3b, 3c) über den Überführungskanal (4) in den zweiten Kultivierungskanal (2a, 2b, 2c) mittels einer zweiten Überführungsflüssigkeit und ein Züchten einer weiteren Zellkultur durch Vermehren der Zellen in dem zweiten Kultivierungskanal (2a, 2b, 2c) umfasst.

8. Substrat (1) zum Kultivieren von Zellen mittels des Verfahrens nach einem der vorangegangenen Ansprüche,
wobei in dem Substrat (1) ein Fluidkanalsystem (1a) ausgebildet ist, das umfasst:
einen Kultivierungskanal (2a, 2b, 2c) zum Züchten einer Zellkultur mit einem Kultivierungszugang (5a, 5b, 5c) nach außen;
Speicherkanal (3a, 3b, 3c) zum Speichern und/oder Konzentrieren und/oder Homogenisieren und/oder Verdünnen einer die überführten Zellen enthaltenden Zellsuspension mit einem Speicherzugang (6a, 6b, 6c) nach außen;
einen Überführungskanal (4) mit einem Überführungszugang (7a, 7b) nach außen,
wobei der Kultivierungskanal (2a, 2b, 2c) und der Speicherkanal (3a, 3b, 3c) fluidisch über den Überführungskanal (4) miteinander verbunden sind.

9. Substrat (1) nach Anspruch 8, wobei das Fluidkanalsystem (1a) derart ausgebildet ist, insbesondere eine derartige Form, Anordnung, Orientierung und/oder Verbindung der Kanäle, aufweist, dass allein durch selektives Öffnen und Schließen der Zugänge und/oder das Anlegen von Druck an das Fluidkanalsystem (1a) Flüssigkeiten auf den für den jeweiligen Verfahrensschritt vorgesehenen Transportwegen durch das Fluidkanalsystem (1a) bewegt werden und/oder mit für den Verfahrensschritt erforderlichen Fließgeschwindigkeiten durch das Fluidkanalsystem (1a) bewegt werden.

10. Substrat (1) nach Anspruch 8 oder 9,
wobei der Kultivierungskanal (2a, 2b, 2c), der Speicherkanal (3a, 3b, 3c) und der Überführungskanal (4) in einer Ebene angeordnet sind und/oder
wobei eine Mündung des Kultivierungskanals (2a, 2b, 2c) in den Überführungskanal (4) entlang des Überführungskanals (4) versetzt zu einer Mündung des Speicherkanals (3a, 3b, 3c) in den Überführungskanal (4) angeordnet ist.

11. Substrat (1) nach einem der Ansprüche 8 bis 10,
wobei die Längsachse des Überführungskanals (4) in einem Winkel größer oder gleich 50°, insbesondere größer oder gleich 55°, insbesondere größer oder gleich 65°, insbesondere größer oder gleich 75°, insbesondere größer oder gleich 85°, insbesondere im Wesentlichen senkrecht zu der jeweiligen Längsachse des Kultivierungskanals (2a, 2b, 2c) angeordnet ist.

12. Substrat (1) nach einem der Ansprüche 8 bis 11, wobei der Kultivierungskanal (2a, 2b, 2c) zu dem Überführungskanal (4) hin verengt ist und/oder wobei der Speicherkanal (3a, 3b, 3c) zu dem Überführungskanal (4) hin verengt ist.

13. Substrat (1) nach einem der Ansprüche 8 bis 12,
wobei mindestens eine Wand des Kultivierungskanals (2a, 2b, 2c), insbesondere mindestens ein Teilbereich des Bodens oder der Decke oder der Seitenwände, eine Oberflächenbeschaffenheit des Materials, insbesondere eine Materialeigenschaft und/oder eine strukturelle Eigenschaft, und/oder eine Beschichtung (2a') aufweist, die das Anwachsen von Zellen fördert, insbesondere eine hydrophile Beschichtung und/oder eine zelladhärierende Beschichtung, und/oder
wobei mindestens eine Wand des Kultivierungskanals (2a, 2b, 2c), insbesondere mindestens ein Teilbereich des Bodens oder der Decke oder der Seitenwände, mit einer Oberflächenmodifizierung versehen ist, die hinsichtlich ihrer physikalischen und/oder chemischen Eigenschaften derart schaltbar ist, dass durch das Schalten eine Ablösung der Zellen von der Oberfläche bewirkt wird, insbesondere mit einer thermoresponsiven Oberflächen-Modifizierung, insbesondere mit einer Beschichtung mit Poly-N-Isopropylacrylamid (PNIPAM- basierten Polymeren, und/oder
wobei mindestens eine Wand des Kultivierungskanals (2a, 2b, 2c), insbesondere mindestens ein Teilbereich des Bodens, mit einer regenerierbaren Oberflächenmodifizierung versehen ist, die insbesondere derart ausgebildet ist, dass die Zelladhäsion veränderbar, aktivierbar und/oder deaktivierbar ist, insbesondere über kompetitive Hemmung veränderbar, und/oder
wobei mindestens eine Wand des Kultivierungskanals (2a, 2b, 2c), insbesondere mindestens ein Teilbereich des Bodens und/oder der Seitenwände, eine Strukturierung aufweist, die derart ausgebildet ist, dass die Wachstumsoberfläche erhöht wird, insbesondere ein Sägezahnprofil und/oder ein Wellenprofil und/oder eine Lamellenstruktur und/oder faserartige und/oder poröse bzw. schwammartige Bereiche aufweist.

14. Substrat (1) nach einem der Ansprüche 8 bis 13, wobei mindestens eine Wand, insbesondere alle Wände, des Überführungskanals (4) und/oder des Speicherkanals (3a, 3b, 3c) eine Oberflächenbeschaffenheit des Materials, insbesondere eine Materialeigenschaft und/oder eine strukturelle Eigenschaft, und/oder eine Beschichtung (3a', 4') aufweisen, die das Anwachsen von Zellen hindert, insbesondere eine hydrophobe Beschichtung und/oder eine zellabweisende Beschichtung.

15. Substrat (1) nach einem der Ansprüche 8 bis 14, wobei der Boden des Speicherkanals (3a, 3b, 3c) eine Strukturierung aufweist, beispielsweise ein Sägezahnprofil und/oder ein Wellenprofil, die derart ausgebildet ist, dass die Bewegung von Zellen, die auf dem Boden liegen, mindestens in einer Richtung erschwert, insbesondere verhindert wird.

16. System umfassend ein Substrat (1) nach einem der Ansprüche 8 bis 15 und mindestens drei außerhalb des Substrats (1) angeordnete Flüssigkeitsbehälter (12a-12d),
wobei der Kultivierungszugang (5a, 5b, 5c), der Speicherzugang (6a, 6b, 6c) und der Überführungszugang (7a, 7b) jeweils mit mindestens einem der Flüssigkeitsbehälter (12a-12d) fluidisch verbunden sind,
aktive Komponenten (13a-13c, 15a-15c, 16a-16d, 17a, 17b, 18a-18c), insbesondere Pumpen und/oder Ventile, die derart ausgebildet und angeordnet sind, dass sie bei entsprechender Betätigung, insbesondere Ansteuerung, eine kontrollierte Zufuhr von Flüssigkeiten aus den Flüssigkeitsbehältern (12a-12d) in das Fluidkanalsystem (1a) und/oder einen kontrollierten Transport der Flüssigkeiten in dem Fluidkanalsystem (1a) und/oder eine kontrollierte Abfuhr von Flüssigkeiten aus dem Fluidkanalsystem (1a) bewirken,
wobei alle aktiven Komponenten (13a-13c, 15a-15c, 16a-16d, 17a, 17b, 18a-18c) außerhalb des Substrats (1) angeordnet sind.

17. System nach Anspruch 16,
wobei die mindestens drei Flüssigkeitsbehälter (12a-12d) zwei Speicherbehälter (12a, 12b, 12c) für Prozessflüssigkeiten und einen Abfallbehälter (12d) umfassen und
wobei der Kultivierungszugang (5a, 5b, 5c) mit einem Ventil (15a, 15b, 15c) verbunden ist, das zum Herstellen einer Fluidverbindung zwischen dem Kultivierungskanal (2a, 2b, 2c) und mindestens einem der Speicherbehälter (12a, 12b, 12c) und zum Herstellen einer Fluidverbindung zwischen dem Kultivierungskanal (2a, 2b, 2c) und dem Abfallbehälter (12d) ausgebildet ist oder wobei der Kultivierungszugang (5a, 5b, 5c) mit zwei Ventilen (15a, 15b, 15c) verbunden ist, von denen eines zum Herstellen einer Fluidverbindung zwischen dem Kultivierungskanal (2a, 2b, 2c) und mindestens einem der Speicherbehälter (12a, 12b, 12c) und das andere zum Herstellen einer Fluidverbindung zwischen dem Kultivierungskanal (2a, 2b, 2c) und dem Abfallbehälter (12d) ausgebildet ist, und/oder
wobei der Speicherzugang (6a, 6b, 6c) mit einem Ventil (16a, 16b, 16c, 16d) verbunden ist, das zum Herstellen einer Fluidverbindung zwischen dem Speicherkanal (3a, 3b, 3c) und dem Abfallbehälter (12d) ausgebildet ist, und/oder
wobei der Überführungszugang (7a, 7b) mit einem Ventil (17a, 17b) verbunden ist, das zum Herstellen einer Fluidverbindung zwischen dem Überführungskanal (4) und dem Abfallbehälter (12d) ist, und insbesondere wobei der Überführungszugang mit dem Ventil (17a, 17b) oder einem weiteren Ventil verbunden ist, wobei das Ventil (17a, 17b) oder das weitere Ventil zum Herstellen einer Fluidverbindung zwischen dem Überführungskanal (4) und mindestens einem der Speicherbehälter (12a, 12b, 12c) ausgebildet ist.

18. System nach einem der Ansprüche 16 oder 17, umfassend eine außerhalb des Substrats (1) angeordnete Messeinrichtung (22a-22d), die zum Messen von Prozess-Messgrößen ausgebildet ist, und/oder eine außerhalb des Substrats (1) angeordnete Messeinrichtung (23), die zur optischen Untersuchung der Zellen im Substrat (1) ausgebildet ist.

19. System nach einem der Ansprüche 16 bis 18,
umfassend eine Steuereinrichtung, die dazu ausgebildet ist, die aktiven Komponenten (13a-13c, 15a-15c, 16a-16d, 17a, 17b, 18a-18c) derart zu steuern, dass ein Verfahren nach einem der Ansprüche 1 bis 7 durchgeführt wird.
wobei die Steuereinrichtung insbesondere derart ausgebildet ist, Werte von an den aktiven Komponenten (13a-13c, 15a-15c, 16a-16d, 17a, 17b, 18a-18c) einzustellenden Parametern basierend auf Soll-Werten und gemessenen Ist-Werten von Prozess-Messgrößen zu regeln, die mittels der Messeinrichtung (22a-22d) zum Messen von Prozess-Messgrößen erfasst werden.
